# EUROPÄISCHE PATENTSCHRIFT

(11) **EP 2 430 059 B1**
(45) Veröffentlichungstag und Bekanntmachung des Hinweises auf die Patenterteilung: **17.09.2014**
(21) Anmeldenummer: 10721442.1
(22) Anmeldetag: 12.05.2010
(51) Int. Cl.: C08F 226/10

(54) **FÄLLUNGSPOLYMERISATE**
PRECIPITATED POLYMERS
POLYMÈRES PRÉCIPITÉS

(30) Priorität: 15.05.2009 EP 09160344
(43) Veröffentlichungstag der Anmeldung: 21.03.2012
(73) Patentinhaber: BASF SE, 67056 Ludwigshafen (DE)
(72) Erfinder: NGUYEN KIM, Son, 69502 Hemsbach (DE); FAST, Ina, 67227 Frankenthal (DE); WERNER, Rolf, 67069 Ludwigshafen (DE)
(74) Vertreter: Reinhardt, Jürgen
(86) Internationale Anmeldenummer: PCT/EP2010/056512
(87) Internationale Veröffentlichungsnummer: WO 2010/130763

(56) Entgegenhaltungen:
- EP-A1- 1 000 610
- WO-A2-2004/058837

## Beschreibung

Die vorliegende Erfindung betrifft Fällungspolymerisate, die 80 bis 99,9 Gew.-% wenigstens eines nichtionischen wasserlöslichen Monomers a) und 0,1 bis 20 Gew.-% eines Amid- oder Harnstoffgruppen tragenden Monomers b) einpolymerisiert enthalten. Die Erfindung betrifft weiterhin die Verwendung dieser Polymerisate als Rheologiemodifizierungsmittel für insbesondere wässrige Zusammensetzungen und als rheologiemodifizierende Festiger in der Haarkosmetik. Die Erfindung betrifft weiterhin wässrige Zusammensetzungen enthaltend diese Polymerisate.

Eine Aufgabe der vorliegenden Erfindung ist die Bereitstellung von Produkten, insbesondere kosmetischen Produkten, mit einem komplexen Eigenschaftsprofil unter Einsatz einer möglichst geringen Menge des jeweiligen Wirkstoffes und/oder einer möglichst geringen Anzahl verschiedener Wirkstoffe.
Im Bereich der Haarkosmetik besteht ein Bedarf an Polymeren, die sowohl eine Einstellung der rheologischen Eigenschaften der Zubereitungen ermöglichen als auch eine festigende Wirkung bei der Anwendung auf den Haaren besitzen.
Eine weitere Aufgabe der vorliegenden Erfindung ist es, Polymere mit einem möglichst hohen Gehalt an einpolymerisiertem N-Vinylpyrrolidon, guten Festigereigenschaften bei der Anwendung im Haar und geringer Klebrigkeit bereitzustellen, die sich zudem vorteilhaft herstellen und weiterverarbeiten lassen.
Häufig werden Polymere bei ihrer Herstellung bzw. der nachfolgenden Verarbeitung erhöhter mechanischer Belastung ausgesetzt. Bei zahlreichen Polymeren, die in den vorgenannten Anwendungsgebieten verwendet werden, führt eine derartige Belastung häufig zu sehr geringen Teilchengrößen, die dann eine unerwünschte Staubbildung bedingen.

EP-A 328725 und EP-A 814101 beschreiben die Herstellung von Acrylsäure-basierten Verdickern durch Fällungspolymerisation.

US 5130388 beschreibt die Herstellung von Acrylsäure-Acrylamid-Copolymeren durch Fällungspolymerisation.

WO 2006/114404 beschreibt ein Verfahren zur Herstellung von Fällungspolymeren durch Sprühpolymerisation einer Monomerlösung, enthaltend mindestens ein ethylenisch ungesättigtes Monomer a), mindestens ein Lösungsmittel b), gegebenenfalls mindestens einen Vernetzer c) und gegebenenfalls mindestens einen Initiator d) wobei Monomer a) im Lösungsmittel b) löslich ist und das durch Polymerisation von a) erhaltene Polymer im Lösungsmittel b) nicht löslich ist.

WO 2007/010035 beschreibt die Verwendung von ampholytischen Copolymeren als Verdicker für kosmetische Zubereitungen, wobei die Copolymere durch Fällungspolymerisation hergestellt werden.

WO 2003/092640 beschreibt Copolymere auf Basis von Vinylpyrrolidon und Methacrylamid, die durch Lösungspolymerisation erhalten werden.

WO 2005/123014 beschreibt Copolymere auf Basis von Vinylpyrrolidon, Vinylimidazol und Methacrylamid, die durch radikalische Lösungspolymerisation erhalten werden.

US 5015708 beschreibt ein Verfahren zur Herstellung eines Terpolymers aus (i) einem Vinyllactam, (ii) einem Säuregruppen-haltigen Monomer und (iii) einem hydrophoben Monomer, wobei es sich u. a. um eine ethylenisch ungesättigte Siliconverbindung handeln kann, durch Fällungspolymerisation sowie die Herstellung von Pulvern aus diesen Polymeren.

WO 04/058837 beschreibt ampholytische Copolymere, die durch radikalische Copolymerisation von
a) wenigstens einer ethylenisch ungesättigten Verbindung mit mindestens einer anionogenen und/oder anionischen Gruppe,
b) wenigstens einer ethylenisch ungesättigten Verbindung mit mindestens einer kationogenen und/oder kationischen Gruppe,
c) wenigstens einer ungesättigten amidgruppenhaltigen Verbindung sowie gegebenenfalls weiterer Comonomere erhältlich sind.

EP 1000610 A1 (BASF) beschreibt Lösungspolymerisate bestehend aus a) 0,05 bis 90 Gew.% Monomeren der Formel 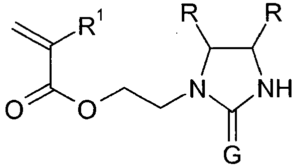 worin R¹ H oder CH₃, G O oder NH und R H oder gleichartige oder ungleichartige organische Reste, die auch miteinander verbunden sein können, bedeuten, und b) 99,95 bis 10 Gew.% Vinylcaprolactam und/oder Vinylpyrrolidon.

Der vorliegenden Erfindung lag die Aufgabe zu Grunde, neue Polymere zur Verfügung zu stellen, die sich zur Modifizierung der rheologischen Eigenschaften von kosmetischen Zusammensetzungen und als Filmbildner eignen und gleichzeitig festigende Wirkung bei der Anwendung auf Haaren besitzen. Insbesondere sollten sich diese Polymere im Wesentlichen ohne Staubentwicklung herstellen und verarbeiten lassen.

Gelförmige Präparate für die Kosmetik sollen möglichst viele der folgenden Eigenschaften in sich vereinen:
- die erhaltenen Gele sollen möglichst klar sein,
- die erhaltenen Gele sollen sich leicht im Haar verteilen lassen und diesem guten Halt verleihen, was sich besonders gut durch Gele mit thixotropen Eigenschaften erzielen lässt,
- die erhaltenen Gele sollen selbst über filmbildende Eigenschaften verfügen und so zur Festigung der Haare beitragen,
- die erhaltenen Gele sollen über konditionierende Eigenschaften verfügen und die sensorischen Eigenschaften des Haars verbessern, z. B. ihm Geschmeidigkeit und Glanz verleihen und nach dem Trocken nicht oder nur gering klebrig sein,
- das mit den erhaltenen Gelen behandelte Haar soll eine gute Nasskämmbarkeit aufweisen (somit lässt sich das frisch behandelte Haar leicht mit dem Kamm in Form bringen, um die gewünschte Frisur zu formen),
- die zur Gelherstellung verwendeten Polymere sollen es erlauben, dass in möglichst allen kosmetisch akzeptablen pH-Bereichen, speziell im pH-Bereich von etwa 5 bis 9 Gele formuliert werden können,
- die zur Gelherstellung verwendeten Polymere sollen die Formulierung von Gelen erlauben, deren Eigenschaften über den pH-Wert schaltbar sind,
- die zur Gelherstellung verwendeten Polymere sollen gemeinsam mit handelsüblichen Verdickern formulierbar sein.

Überraschend wurde gefunden, dass diese Aufgaben gelöst werden durch Polymerisate erhältlich durch Fällungspolymerisation, gemäss Anspruch 1.

Im Folgenden werden die erfindungsgemäßen Polymerisate, die durch Fällungspolymerisation erhältlich sind, auch als "Fällungspolymerisate" bezeichnet. Im Folgenden sind die Begriffe "Polymerisate" und "Polymere" gleichbedeutend.

Ohne sich auf die Beschreibung der Formel (I) zu beschränken, umfasst der Ausdruck Alkyl im Rahmen der vorliegenden Erfindung geradkettige und verzweigte Alkylgruppen. Geeignete kurzkettige Alkylgruppen sind z. B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 1-Methylbutyl, 2-Methyl-butyl, 3-Methylbutyl, 1,2-Dimethylpropyl, 1,1-Dimethylpropyl, 2,2-Dimethylpropyl, Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl.

Ohne sich auf die Beschreibung der Formel (I) zu beschränken, umfasst der Ausdruck C₈-C₃₀-Alkyl- bzw. C₈-C₃₀-Alkenylgruppen im Rahmen dieser Erfindung generell geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en), Arachinyl(en), Behenyl(en), Ligno-cerinyl(en), Melissinyl(en). Bevorzugt sind auch verzweigte C₈-C₃₀-Alkylreste wie beispielsweise 2-Ethyl-Hexyl.

Ohne sich auf die Beschreibung der Formel (I) zu beschränken, umfasst der Ausdruck Cycloalkyl im Rahmen dieser Erfindung vorzugsweise C₅-C₈-Cycloalkyl, wie Cyclopentyl, Cyclohexyl, Cycloheptyl oder Cyclooctyl.

Ohne sich auf die Beschreibung der Formel (I) zu beschränken, umfasst der Ausdruck Aryl im Rahmen dieser Erfindung generell unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Unter wasserlöslichen Monomeren bzw. wasserlöslichen Polymeren werden im Rahmen der vorliegenden Erfindung solche Monomere bzw. Polymere verstanden, die sich bei 20°C zu mindestens 1 Gramm pro Liter (g/I), bevorzugt zu mindestens 10 g/l in Wasser derart lösen, dass sich eine für das menschliche Auge klare Lösung ergibt.

Unter wasserdispergierbaren Monomeren bzw. wasserdispergierbaren Polymeren werden im Rahmen dieser Erfindung generell solche Monomere bzw. Polymere verstanden, die unter Anwendung von Scherkräften, beispielsweise durch Rühren, in wasserdispergierbare Partikel zerfallen. Hydrophile Monomere sind vorzugsweise wasserlöslich oder zumindest wasserdispergierbar. Die erfindungsgemäßen Fällungspolymerisate sind im Allgemeinen wasserlöslich oder wasserdispergierbar, bevorzugt sind sie wasserlöslich.

Monomere a) sind N-Vinyllactame und deren Derivate, die z.B. einen oder mehrere C₁-C₆-Alkylsubstituenten, wie Methyl, Ethyl, n-Propyl, Isopropyl, n-Butyl, sec.-Butyl, tert.-Butyl aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam.

Besonders bevorzugt werden N-Vinylpyrrolidon und/oder N-Vinylcaprolactam als Monomer a) eingesetzt.

Die erfindungsgemäßen Fällungspolymerisate enthalten bevorzugt wenigstens 92 Gew.-%, weiter bevorzugt wenigstens 94 Gew.-% und bevorzugt höchstens 98, weiter bevorzugt höchstens 96 Gew.-% Monomer a) einpolymerisiert.

### Monomer b)

Erfindungsgemäß ist Monomer b) ausgewählt aus
b1) Methacrylamid,
b2) radikalisch polymerisierbaren Verbindungen, die ein Strukturelement der Formel b2) enthalten 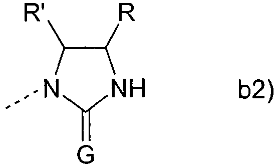 worin R und R'unabhängig voneinander Wasserstoff, Alkyl, Cycloalkyl, Heterocycloalkyl, Aryl oder Hetaryl, wobei R und R' auch kovalent verbunden sein können, und G für O oder NH steht,
b3) wenigstens eine Allylaminogruppe tragenden Polyhydroxyverbindungen und
b4) deren Mischungen.

Eine Ausführungsform der Erfindung sind erfindungsgemäße Fällungspolymerisate, die als Monomer b) b1) Methacrylamid einpolymerisiert enthalten.

Eine weitere Ausführungsform der Erfindung sind erfindungsgemäße Fällungspolymerisate, die als Monomer b) b2) wenigstens eine radikalisch polymerisierbare Verbindung, die ein Strukturelement der Formel b2) enthält 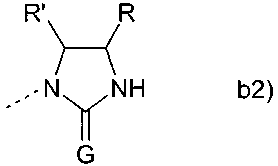 worin R und R'unabhängig voneinander Wasserstoff, Alkyl, Cycloalkyl, Heterocycloalkyl, Aryl oder Hetaryl, wobei R und R' auch kovalent verbunden sein können, und G für O oder NH steht, einpolymerisiert enthalten.
Geeignete Monomere b) sind in EP 1000610 A1 (BASF) beschrieben, insbesondere in Absatz [0011], worauf hiermit Bezug genommen wird.
Besonders bevorzugt als Monomer b2) ist die verbindung der folgenden Formel b2a) 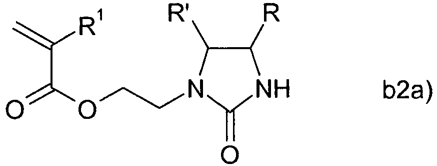 worin R, R' und H wie oben definiert und R¹ Wasserstoff oder Methyl bedeutet.
In einer bevorzugten Ausführungsform der Erfindung ist Monomer b2) 2-Ethyl-(2-oxoimidazolidin-1-yl)methacrylat, also R und R' sind Wasserstoff und R¹ ist Methyl.
2-Ethyl-(2-oxoimidazolidin-1-yl)methacrylat wird im Rahmen dieser Erfindung auch als "Ureidomethacrylat" oder kurz "UMA" bezeichnet.
Eine bevorzugte Ausführungsform der Erfindung sind Fällungspolymerisate, die von 0,1 bis 5, bevorzugt von 0,5 bis 3 Gew.-% Ureidomethacrylat einpolymerisiert enthalten.
Ureidomethacrylat wird bevorzugt als Mischung mit Methylmethacrylat eingesetzt, wobei solche Mischungen bevorzugt im Bereich von 20 bis 60, bevorzugt von 25 bis 50 Gew.-% UMA enthalten. Kommerziell erhältlich sind solche Mischungen beispielsweise als Norsocryl^{®} (Arkema), Plex^{®}6844-O (Röhm) oder 25% UMA in Methylmethacrylat MMA (BASF).

Eine weitere Ausführungsform der Erfindung sind erfindungsgemäße Fällungspolymerisate, die als Monomer b3) eine wenigstens eine Allylaminogruppe tragende Polyhydroxyverbindung einpolymerisiert enthalten.

Unter einer Allylaminogruppe wird im Rahmen der vorliegenden Erfindung bevorzugt die Struktureinheit -NR⁵-CH=CH₂ verstanden, wobei R⁵ Wasserstoff oder C₁-C₄-Alkyl, besonders bevorzugt Wasserstoff bedeutet.

Bevorzugte vorgenannte Polyhydroxyverbindungen sind mindestens drei OH-Gruppen enthaltende Verbindungen aus der Gruppe bestehend aus Polyglycerin, Zuckercarbonsäuren, Alkylglucoside, Mono- und Oligosaccharide, die bis zu vier Monosaccharideinheiten enthalten, Zuckeralkohole und Oxidationsprodukte der genannten Oligosaccharide, Aminosorbit, Aminodisorbit, Glucosamin, N-Acetylglucosamin, Triethanolamin und Trishydroxyethylmelamin, wobei diese Verbindungen jeweils wenigstens eine Allylaminogruppe aufweisen.

Bevorzugte Polyglycerine sind solche, die 3 bis 10 Glycerin-Einheiten enthalten. Solche Verbindungen entstehen beispielsweise bei der Kondensation von Glycerin in Gegenwart von Alkali oder Säure. Unter Polyglycerin im Sinne der vorliegenden Erfindung sollen auch die Oligomere verstanden werden wie Triglycerin, Tetraglycerin, Pentaglycerin und Hexaglycerin sowie Polymere, die bis zu 10 Glycerin-Einheiten im Molekül aufweisen, wobei alle diese Verbindungen jeweils wenigstens eine Allylaminogruppe aufweisen.

Als Zuckercarbonsäuren kommen bevorzugt die Oxidationsprodukte von Zuckern mit 4 bis 7 Kohlenstoffatomen in Betracht, z. B. Gluconsäure, Glucoheptonsäure, Glucarsäure, Galactarsäure, Glucuronsäure oder Mannonsäure sowie die entsprechenden Lactone, z. B. Gluconolacton und Glucoheptonolacton, wobei diese Verbindungen jeweils wenigstens eine Allylaminogruppe aufweisen.

Weitere geeignete Polyhydroxyverbindungen sind jeweils wenigstens eine Allylaminogruppe tragende Alkylglucoside und Alkylpolyglucoside, Alkylmaltoside und Alkylmaltotrioside. Die Alkylgruppe kann C₁ bis C₅, vorzugsweise eine C₁- bis C₄-Alkylgruppe sein, z. B. Methyl, Ethyl, n-Propyl, Isopropyl, n-Butyl oder Isobutyl. Außerdem kann die Alkylgruppe substituiert sein, z. B. eine Hydroxylgruppe tragen. Geeignete Verbindungen dieser Art sind beispielsweise Hydroxyethylglucosid und Hydroxypropylglucosid sowie die entsprechenden Polyglucoside. Die Polyglucoside enthalten im Mittel 1,1 bis 10, vorzugsweise 1,3 bis 3 Glucosideinheiten.

Geeignete wenigstens eine Allylaminogruppe tragende Oligosaccharide, die bis zu 4 Monosaccharideinheiten enthalten, sind beispielsweise wenigstens eine Allylaminogruppe tragende Maltose, Maltotriose, Maltotetraose, Saccharose, Lactose, Leucrose, Isomaltulose, Chitobiose, Chitotriose, Chitotetraose und die davon durch Abspalten der Acetylgruppen erhältlichen Derivate. Als Monosaccharideinheiten der Oligosaccharide kommen alle üblichen Monosaccharide in Betracht, insbesondere von Glucose, Galactose, Fructose und Mannose abgeleitete Einheiten.

Zuckeralkohole von Oligosacchariden, die bis zu 4 Monosaccharideinheiten enthalten, sind aus den obengenannten Oligosacchariden durch Reduktion erhältlich. Zu den O-xidationsprodukten der genannten Oligosaccharide gehört beispielsweise Saccharosetricarbonsäure und Lactobionsäure.

Weitere geeignete wenigstens eine Allylaminogruppe tragende Polyhydroxyverbindungen sind außerdem wenigstens eine Allylaminogruppe tragende Monosaccharide wie z. B. Glucose, Galactose, Mannose, und Fructose, die jeweils wenigstens eine Allylaminogruppe aufweisen.

Weitere geeignete wenigstens eine Allylaminogruppe tragende Polyhydroxyverbindungen sind Aminosorbit, Aminodisorbit, Glucosamin, N-Acetylglucosamin, Triethanolamin und Trishydroxyethylmelamin, die jeweils wenigstens eine Allylaminogruppe tragen.

Bevorzugte geeignete wenigstens eine Allylaminogruppe tragende Polyhydroxyverbindungen sind Polyglycerine mit 3 bis 10 Glycerin-Einheiten, Gluconsäure, Glucoheptonsäure, Maltose und Hydroxyethylglucosid, die jeweils wenigstens eine Allylaminogruppe tragen.

Bevorzugte wenigstens eine Allylaminogruppe tragende Polyhydroxyverbindungen als Monomere b) sind die Allylamide von Zuckercarbonsäuren.

Besonders bevorzugte wenigstens eine Allylaminogruppe tragende Polyhydroxyverbindungen als Monomere b) sind Allylamide von Zuckercarbonsäuren, wobei die Zuckercarbonsäure ausgewählt ist aus der Gruppe bestehend aus den Oxidationsprodukte von Zuckern mit 4 bis 7 Kohlenstoffatomen. Solche Zuckercarbonsäuren sind beispielsweise Gluconsäure, Glucoheptonsäure, Glucarsäure, Galactarsäure, Glucuronsäure oder Mannonsäure.

Die besonders bevorzugte Allylaminogruppen tragende Polyhydroxyverbindung als Monomer b) ist Allyl-D-Gluconamid der Formel II

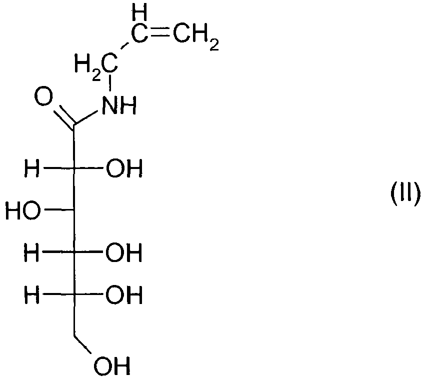

Eine weitere Ausführungsform der Erfindung sind also erfindungsgemäße Fällungspolymerisate, die als Monomer b) Allyl-D-Gluconamid einpolymerisiert enthalten.

### Weitere Monomere

Die erfindungsgemäßen Fällungspolymerisate können von 0 Gew.-% bis 19,9 Gew.-%, bevorzugt von 0,5 bis 15 Gew.-% weitere Monomere c) einpolymerisiert enthalten, die von a) und b) verschieden sind.
Maßgeblich ist, dass die Gesamtmenge aller in die erfindungsgemäßen Fällungspolymerisate einpolymerisierten Monomere 100 Gew.-% beträgt.

### c1) Anionische oder anionogene Monomere

Bevorzugte weitere Monomere c) sind Verbindungen c1) mit einer radikalisch polymerisierbaren, α, β -ethylenisch ungesättigten Doppelbindung und wenigstens einer anionogenen und/oder anionischen Gruppe pro Molekül. Säuregruppen enthaltende Monomere werden im ungeladenen Zustand als anionogen, im geladenen Zustand als anionisch bezeichnet.
Eine Ausführungsform der Erfindung sind also erfindungsgemäße Fällungspolymerisate, die wenigstens 0 Gew.-% bis 19,9 Gew.-% eines Monomer c1) einpolymerisiert enthalten, wobei die Summe aller einpolymerisierten Monomere 100 Gew.-% beträgt. Bevorzugte weitere Monomere c) sind Verbindungen mit einer radikalisch polymerisierbaren, α, β -ethylenisch ungesättigten Doppelbindung und wenigstens einer anionogenen und/oder anionischen Gruppe pro Molekül. Säuregruppen enthaltende Monomere werden im ungeladenen Zustand als anionogen, im geladenen Zustand als anionisch bezeichnet.
Bevorzugte Verbindungen c1) sind Verbindungen, die ausgewählt sind unter monoethylenisch ungesättigten Carbonsäuren, Sulfonsäuren, Phosphonsäuren und Mischungen davon. Dazu zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Verbindungen c1) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Verbindungen c1) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Verbindungen c1) zählen auch die Salze der zuvor genannten Säuren, insbesondere die Natrium-, Kalium- und Ammoniumsalze sowie die Salze mit Aminen. Diese Monomere c1) können als solche oder als Mischungen eingesetzt werden. Bevorzugte Monomere c1) sind ausgewählt aus der Gruppe bestehend aus Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure, 2-Acrylamido-2-methylpropansulfonsäure, Vinylphosphonsäure und Mischungen davon.
Besonders bevorzugt wird Monomer c1) ausgewählt aus der Gruppe bestehend aus Acrylsäure, Methacrylsäure und deren Mischungen.
In einer Ausführungsform der Erfindung enthalten die erfindungsgemäßen Polymerisate Acrylsäure einpolymerisiert. In einer bevorzugten Ausführungsform der Erfindung enthalten die erfindungsgemäßen Polymerisate von 1 bis 15 Gew.-%, weiter bevorzugt von 2 bis 10 Gew.-% Acrylsäure einpolymerisiert.

### c2) Vinylimidazole

Eine Ausführungsform der Erfindung sind erfindungsgemäße Fällungspolymerisate, die von 0 Gew.-% bis 19,9 Gew.-%, bevorzugt von 0,5 bis 15 Gew.-% wenigstens eines Monomer c2) einpolymerisiert enthalten, wobei die Summe aller einpolymerisierten Monomere 100 Gew.-% beträgt.
Bevorzugte Monomere c2) sind Monomere der allgemeinen Formel IIa 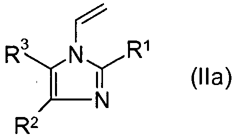 wobei R¹ bis R³ unabhängig voneinander Wasserstoff, C₁-C₄-Alkyl oder Phenyl bedeuten.

Beispiele für Monomere c2) sind folgender Tabelle 1 zu entnehmen:

**Tabelle 1**

| R¹ | R² | R³ |
|---|---|---|
| | | |
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

Besonders bevorzugte Monomere c2) sind 1-Vinylimidazol (N-Vinylimidazol) und Mischungen, die N-Vinylimidazol enthalten.
Eine Ausführungsform der Erfindung sind erfindungsgemäße Polymerisate, die von 2 bis 15, bevorzugt von 5 bis 10 Gew.-% 1-Vinylimidazol einpolymerisiert enthalten, wobei die Summe aller einpolymerisierten Monomere 100 Gew.-% beträgt.

### c3) am Stickstoff C₁-C₂₄-Alkyl substituierte Ester der (Meth)acrylsäure

Bevorzugte weitere Monomere c) sind c3) am Stickstoff ein- oder zweifach C₁-C₂₄-Alkyl substituierte Ester der (Meth)acrylsäure mit Aminoalkoholen (Monomere c3). Besonders bevorzugt sind die Monomere c3) ausgewählt aus der Gruppe bestehend aus N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.
Besonders bevorzugt als Monomer c3) ist N,N-Dimethylaminoethylmethacrylat (DMAEMA).
Eine Ausführungsform der Erfindung sind also erfindungsgemäße Fällungspolymerisate, die wenigstens ein Monomer c3) einpolymerisiert enthalten.

Eine weitere Ausführungsform der Erfindung sind erfindungsgemäße Polymere, die von 2 bis 15, bevorzugt von 5 bis 10 Gew.-% DMAEMA einpolymerisiert enthalten, wobei die Summe aller einpolymerisierten Monomere 100 Gew.-% beträgt.

### c4) am Stickstoff C₁-C₂₄-Alkyl substituierte Amide der (Meth)acrylsäure

Bevorzugte weitere Monomere c) sind c4) am Stickstoff ein- oder zweifach C₁-C₂₄-Alkyl substituierte Amide der (Meth)acrylsäure mit Diaminen (c4). Besonders bevorzugt sind die Monomere c4) ausgewählt aus der Gruppe bestehend aus N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)- butyl]methacrylamid, N-[2-(diethylamino)ethyl]acrylamid, N-[4-(dimethylamino)cyclohexyl]acrylamid, N-[4-(dimethylamino)cyclohexyl]methacrylamid, N-[8-(Dimethylamino)octyl]methacrylamid, N-[12-(Dimethylamino)dodecyl]methacrylamid, N-[3-(Diethylamino)propyl]methacrylamid und N-[3-(Diethylamino)propyl]acrylamid. Besonders bevorzugtes Monomer c4) ist N-[3-(dimethylamino)propyl]methacrylamid (DMAPMAM).
Eine Ausführungsform der Erfindung sind also erfindungsgemäße Fällungspolymerisate, die wenigstens ein Monomer c4) einpolymerisiert enthalten.
Eine weitere Ausführungsform der Erfindung sind erfindungsgemäße Polymere, die von 2 bis 15, bevorzugt von 5 bis 10 Gew.-% DMAPMAM einpolymerisiert enthalten, wobei die Summe aller einpolymerisierten Monomere 100 Gew.-% beträgt.

### c5) N,N-Diallylamine und N,N-Diallyl-N-alkylamine

Bevorzugte weitere Monomere c) sind c5) N,N-Diallylamine und N,N-Diallyl-N-alkylamine und deren Säureadditionssalze und Quaternisierungsprodukte (Monomere c5)). Alkyl steht dabei vorzugsweise für C₁-C₂₄-Alkyl. Bevorzugte Monomere c5) sind N,N-Diallyl-N-methylamin und N,N-Diallyl-N,N-dimethylammonium-Verbindungen, wie z. B. die Chloride und Bromide. Zu den bevorzugten Monomeren c5) zählt insbesondere N,N-Diallyl-N-methylamin sowie dessen methyliertes Derivat N,N-Diallyl-N,N-Dimethylammoniumchlorid (DADMAC).
Eine Ausführungsform der Erfindung sind also erfindungsgemäße Fällungspolymerisate, die wenigstens ein Monomer c5) einpolymerisiert enthalten, wobei die Summe aller einpolymerisierten Monomere 100 Gew.-% beträgt.
Eine weitere Ausführungsform der Erfindung sind erfindungsgemäße Polymere, die von 2 bis 15, bevorzugt von 5 bis 10 Gew.-% DADMAC einpolymerisiert enthalten, wobei die Summe aller einpolymerisierten Monomere 100 Gew.-% beträgt.

Die Monomere der Gruppen c2) bis c5) wie beispielsweise VI, DMAPMAM, DMAEMA oder Diallyl-N-methylamin werden üblicherweise im ungeladenen Zustand als kationogen, im geladenen Zustand als kationisch bezeichnet. Im Rahmen dieser Erfindung wird teilweise anstelle des Begriffs "kationogen" auch der Begriff "kationisch" verwendet.

### c6) offenkettige N-Vinylamidverbindungen

Bevorzugte weitere Monomere c) sind beispielsweise offenkettige N-Vinylamidverbindungen c6) wie beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, Vinyl-N-methylpropionamid, N-Vinyl-butyramid und Mischungen davon.
Eine Ausführungsform der Erfindung sind also erfindungsgemäße Fällungspolymerisate, die wenigstens ein Monomer c6) einpolymerisiert enthalten, wobei die Summe aller einpolymerisierten Monomere 100 Gew.-% beträgt.

### c7) Ester und Amide α,β-ethylenisch ungesättigter Mono- und Dicarbonsäuren

Bevorzugte Monomere c) sind auch c7) Ester α,β-ethylenisch ungesättigter Mono- und Dicarbonsäuren mit C₁-C₃₀-Alkanolen, bevorzugt C₁-C₂₂-Alkanolen. Bevorzugte Monomere c7) sind weiterhin Amide α,β-ethylenisch ungesättigter Mono- und Dicarbonsäuren mit Mono- und Dialkylaminen, die 1 bis 30 Kohlenstoffatome, bevorzugt 1 bis 22 Kohlenstoffatome, pro Alkylrest aufweisen. Vorzugsweise handelt es sich bei den Monomeren c7) um Verbindungen der allgemeinen Formel III 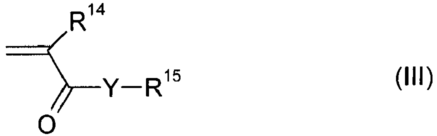 worin
- R¹⁴: für Wasserstoff oder C₁- bis C₈-Alkyl steht,
- R¹⁵: für einen geradkettigen oder verzweigten C₁- bis C₃₀-Alkylrest steht, und
- Y: für O oder NR¹⁶ steht, wobei R¹⁶ für Wasserstoff, C₁- bis C₈-Alkyl oder C₅- bis C₈-Cycloalkyl steht.

Vorzugsweise steht in der Formel III R¹⁴ für Wasserstoff, Methyl oder Ethyl.
Bevorzugt steht Y für O oder NH.
Geeignete Reste R¹⁵ sind die zuvor genannten C₁-C₃₀-Alkylreste. Insbesondere steht R⁵ für Methyl, Ethyl, n-Propyl, Isopropyl, n-Butyl, 2-Butyl, sec.-Butyl, tert.-Butyl, n-Pentyl, n-Hexyl, n-Heptyl, n-Octyl, Ethylhexyl, 1,1,3,3-Tetramethylbutyl, Undecyl, Lauryl, Tridecyl, Myristyl, Pentadecyl, Palmityl, Margarinyl, Stearyl, Palmitoleinyl, Oleyl oder Linolyl.

Insbesondere ist Monomer c7) ausgewählt unter Methyl(meth)acrylat, Methylethacrylat, Ethyl(meth)acrylat, Ethylethacrylat, tert.-Butyl(meth)acrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat, tert.-Butyl(meth)acrylamid, N,N-Dimethyl(meth)acrylamid, n-Octyl(meth)acrylamid, 1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid und Mischungen davon.

Eine weitere Ausführungsform der Erfindung sind erfindungsgemäße Polymere, die von 1 bis 15 Gew.-% Methylmethacrylat einpolymerisiert enthalten, wobei die Summe aller einpolymerisierten Monomere 100 Gew.-% beträgt.

Weitere geeignete Monomere c7) sind Hydroxy-substituierte (Meth)acrylsäureester und Hydroxy-substituierte Methacrylsäureamide, also Verbindungen der Formel III, wobei R¹⁵ für einen geradkettigen oder verzweigten Hydroxysubstituierten C₁- bis C₃₀-Alkylrest steht.
Zu den geeigneten Hydroxy-substituierten (Meth)acrylsäureestern c7) zählen beispielsweise 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.
Zu den geeigneten Hydroxy-substituierten (Meth)acrylsäureamiden c7) zählen beispielsweise 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid,
4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.
Eine Ausführungsform der Erfindung sind also erfindungsgemäße Fällungspolymerisate, die wenigstens ein Monomer c7) einpolymerisiert enthalten, wobei die Summe aller einpolymerisierten Monomere 100 Gew.-% beträgt.

### Verbindungen c8)

Eine Ausführungsform der Erfindung sind erfindungsgemäße Fällungspolymerisate, die wenigstens ein Monomer c8) einpolymerisiert enthalten. Verbindungen c8) sind ausgewählt ist unter Verbindungen der allgemeinen Formeln c8-1) und c8-2) 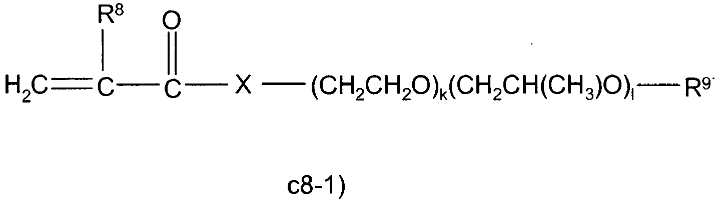 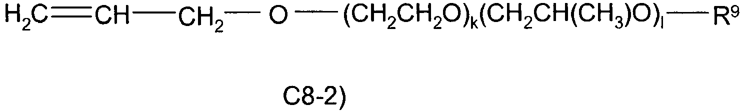 worin
die Reihenfolge der Alkylenoxideinheiten beliebig ist,
- k und I: unabhängig voneinander für eine ganze Zahl von 0 bis 1000 stehen, wobei die Summe aus k und I mindestens 5 beträgt,
- R⁸: für Wasserstoff oder C₁-C₄-Alkyl, vorzugsweise Methyl, steht,
- R⁹: für C₈-C₃₀-Alkyl oder C₈-C₃₀-Alkenyl steht, und
- X: für O oder eine Gruppe der Formel NR¹⁰ steht, worin R¹⁰ für H, Alkyl, Alkenyl, Cycloalkyl, Heterocycloalkyl, Aryl oder Hetaryl steht.

In den Formeln c8-1) und c8-2) steht k vorzugsweise für eine ganze Zahl von 1 bis 500, insbesondere 3 bis 250. Bevorzugt steht I für eine ganze Zahl von 0 bis 100.

Bevorzugt steht R⁸ in der Formel c8-1) für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R⁹ in den Formeln c8-1) und c8-2) für n-Octyl, 1,1,3,3-Tetramethylbutyl, Ethylhexyl, n-Nonyl, n-Decyl, n-Undecyl, Tridecyl, Myristyl, Pentadecyl, Palmityl, Heptadecyl, Octadecyl, Nonadecyl, Arrachinyl, Behenyl, Lignocerenyl, Cerotinyl, Melissinyl, Palmitoleinyl, Oleyl, Linolyl, Linolenyl, Stearyl, Lauryl.

Vorzugsweise steht X in der Formel c8-1) für O oder NH.

Geeignete Polyetheracrylate der Formel c8-1) sind z. B. die Polykondensationsprodukte der zuvor genannten α, β -ethylenisch ungesättigten Mono- und/oder Dicarbonsäuren und deren Säurechloriden, -amiden und -anhydriden mit Polyetherolen. Geeignete Polyetherole können leicht durch Umsetzung von Ethylenoxid, 1,2-Propylenoxid und/oder Epichlorhydrin mit einem Starteralkohol R⁹-OH hergestellt werden. Die Alkylenoxide können einzeln, alternierend nacheinander oder als Mischung eingesetzt werden. Die Polyetheracrylate der Formel c8-1) können allein oder in Mischungen zur Herstellung der erfindungsgemäßen Fällungspolymerisate verwendet werden. Bevorzugte Polyetheracrylate der Formel c8-1) sind Ester der Methacrylsäure mit ethoxylierten C₁₆-C₂₂-Fettatkoholgemischen. Besonders bevorzugt sind Ester der Methacrylsäure mit ethoxylierten C₁₆-C₁₈-Fettalkoholgemischen, wobei der Ethoxylierungsgrad im ungefähr 25 beträgt.
Bevorzugte Polyetheracrylate sind Verbindungen der Formel c8-1), wobei R⁸ Methyl, X Sauerstoff, R⁹ C₁₆-C₁₈-Alkyl, k=25 und I=Null bedeuten: C₁₆-C₁₈-Alkyl-PEG₁₁₀₀-Methacrylat.

Geeignete Allylalkoholalkoxilate c8-2) sind z. B. die Veretherungsprodukte von Allylchlorid mit entsprechenden Polyetherolen. Geeignete Polyetherole können leicht durch Umsetzung von Ethylenoxid, 1,2-Propylenoxid und/oder Epichlorhydrin mit einem Starteralkohol R⁹-OH hergestellt werden. Die Alkylenoxide können einzeln, alternierend nacheinander oder als Mischung eingesetzt werden. Die Allylalkoholalkoxilate c8-2) können allein oder in Mischungen zur Herstellung der erfindungsgemäßen Polymere verwendet werden.

Eine Ausführungsform der Erfindung sind erfindungsgemäße Polymere, die von 0,1 bis 10, bevorzugt von 0,5 bis 3 Gew.-% einer Verbindung c8), bevorzugt der Formel c8-1) einpolymerisiert enthalten, wobei die Summe aller einpolymerisierten Monomere 100 Gew.-% beträgt.

### c9) Urethan(meth)acrylate

Geeignete Monomere c) sind auch c9) Urethan(meth)acrylate wie beispielsweise in der DE-A 198 38 852 S.3, Z.45 bis S.9, Z.20 beschrieben, worauf hier in vollem Umfang Bezug genommen wird. Die dort mit d) bezeichnete Si-Komponente ist allerdings nicht zwingend Bestandteil derjenigen Urethan(meth)acrylate, die im Rahmen der vorliegenden Erfindung als Monomer c9) eingesetzt werden können.
Eine weitere Ausführungsform der Erfindung sind also erfindungsgemäße Fällungspolymerisate, die wenigstens ein Monomer c9) einpolymerisiert enthalten, wobei die Summe aller einpolymerisierten Monomere 100 Gew.-% beträgt.

Geeignete weitere Monomere c) sind Ethylen, Propylen, Isobutylen, Butadien, Styrol, α-Methylstyrol, (Meth)acrylnitril, Vinylchlorid, Vinylidenchlorid, Vinylfluorid, Vinylidenfluorid und Mischungen davon.

Die zuvor genannten zusätzlichen Monomere c) können jeweils einzeln oder in Form von beliebigen Mischungen eingesetzt werden.

### Fällungspolymerisation

Ein weiterer Gegenstand der vorliegenden Erfindung ist ein Verfahren zur Herstellung der erfindungsgemäßen Polymerisate, dadurch gekennzeichnet, dass das Verfahren eine Fällungspolymerisation umfasst.

Bei der Fällungspolymerisation sind die eingesetzten Monomere im Reaktionsmedium, das die Monomere und das Lösungsmittel umfasst, löslich, nicht aber das entstehende Polymer. Das entstehende Polymer wird unter den gewählten Polymerisationsbedingungen unlöslich und fällt aus. Dabei lassen sich Copolymere mit höheren Molekulargewichten erhalten als nach anderen Polymerisationsverfahren, z. B. durch Lösungspolymerisation. Solche Copolymere mit höheren Molekulargewichten eignen sich besonders vorteilhaft als Rheologiemodifizierer, insbesondere als Verdicker.

Die Fällungspolymerisation erfolgt bevorzugt in einem Lösungsmittel, in dem jedes der eingesetzten Monomere bei 20°C und 1 bar in einer Menge von wenigstens 10 Gew.-% für das menschliche Auge klar löslich ist.

Im Rahmen der vorliegende Erfindung gilt ein Polymer in einer flüssigen Phase als unlöslich, wenn weniger als 1 Gramm, bevorzugt weniger als 0,1 Gramm des Polymers in einem Liter der flüssigen Phase für das menschliche Auge klar löslich sind.

Die Fällungspolymerisation erfolgt vorzugsweise in einem weitgehend wasserfreien, aprotischen Lösungsmittel oder Lösungsmittelgemisch. Unter einem weitgehend wasserfreien, aprotischen Lösungsmittel oder Lösungsmittelgemisch wird ein Lösungsmittel oder Lösungsmittelgemisch mit einem Wassergehalt von höchstens 5 Gew.-% verstanden.
Solche aprotischen Lösungsmittel oder Lösungsmittelgemische sind vorzugsweise Ester wie Ethylacetat und/oder n-Butylacetat und/oder Kohlenwasserstoffe wie Cyclohexan oder n-Heptan.
In einer Ausführungsform der Erfindung erfolgt die Fällungspolymerisation in einem Lösungsmittel bestehend aus oder umfassend Ethylacetat.
In einer Ausführungsform der Erfindung erfolgt die Fällungspolymerisation in einem Lösungsmittel bestehend aus oder umfassend n-Butylacetat.
In einer weiteren Ausführungsform der Erfindung erfolgt die Fällungspolymerisation in einem Lösungsmittelgemisch aus wenigstens einem Ester und wenigstens einem Kohlenwasserstoff. Bevorzugtes Lösungsmittelgemisch ist ein Gemisch enthaltend oder bestehend aus 80-90 Gew.-% Ethylacetat und 10-20 Gew.-% Cyclohexan.

Bevorzugt erfolgt die Fällungspolymerisation bei einer Temperatur im Bereich von 70 bis 140 °C, bevorzugt 75 bis 100 °C, insbesondere von 80 bis 95 °C. Die resultierenden Polymerteilchen fallen aus der Reaktionslösung aus und können durch übliche Verfahren, wie Filtration mittels Unterdruck, isoliert werden. Zur Fällungspolymerisation können oberflächenaktive, polymere Verbindungen, vorzugsweise auf Polysiloxanbasis, eingesetzt werden. Bei der Fällungspolymerisation werden in der Regel Polymere mit höheren Molekulargewichten als bei der Lösungspolymerisation erhalten.

Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

### Initiatoren

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, tert.-Butylperoctoat, Azo-bis-isobutyronitril, Azo-bis-(2-amidonopropan)dihydrochlorid, 2,2'-Azo-bis-(2-methyl-butyronitril) oder 2,2'-Azobis (2.4-dimethylvaleronitrile) (Wako^{®}V65). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/ Natriumhydroxymethansulfinat, H₂O₂/Cu(I).

In einer Ausführungsform der Erfindung werden zur Herstellung der erfindungsgemäßen Polymere wenigstens zwei Radikalinitiatoren eingesetzt, deren Zerfallstemperaturen und/oder deren Halbwertszeiten bei einer bestimmten Polymerisationstemperatur voneinander verschieden sind. Dabei können Copolymere mit besonders geringen Restmonomergehalten erzielt werden. Dies ist insbesondere der Fall, wenn der bei höherer Temperatur zerfallende Initiator vor Abschluss der Fällung des Polymers, vorzugsweise vor Beginn der Fällung des Polymers, zugegeben wird.

Bevorzugt werden zur Copolymerisation wenigstens zwei Initiatoren eingesetzt, deren Zerfallstemperaturen um wenigstens 10°C voneinander verschieden sind. Im Rahmen der Erfindung ist die Zerfallstemperatur ist definiert als die Temperatur, bei der 50 % der Moleküle innerhalb von 2,5 Stunden in freie Radikale zerfallen. Vorzugsweise erfolgt die Copolymerisation bei dieser Vorgehensweise bis zum Abschluss der Fällung des Copolymers bei einer Temperatur größer oder gleich der niedrigeren Zerfallstemperatur und kleiner der höheren Zerfallstemperatur, und nach der Fällung erfolgt eine weitere Umsetzung bei einer Temperatur größer oder gleich der höheren Zerfallstemperatur.

Bevorzugt umfasst das erfindungsgemäße Verfahren eine erste Polymerisationsphase bei einer ersten Polymerisationstemperatur und eine zweite Polymerisationsphase bei einer zweiten Polymerisationstemperatur oberhalb der ersten Polymerisationstemperatur, wobei zur Polymerisation wenigstens zwei Initiatoren eingesetzt werden, deren Halbwertszeiten bei der ersten Polymerisationstemperatur sich so unterscheiden, dass wenigstens einer dieser Initiatoren während der ersten Polymerisationsphase in Radikale zerfällt und wenigstens einer dieser Initiatoren während der ersten Polymerisationsphase im Wesentlichen nicht in Radikale zerfällt und während der zweiten Polymerisationsphase in Radikale zerfällt. Vorzugsweise beginnt bei dieser Vorgehensweise die zweite Polymerisationsphase im Wesentlichen nach Fällung des Copolymers. Unter "im Wesentlichen" nach Fällung des Copolymers wird verstanden, dass das Copolymer vorzugsweise zu mindestens 80 Gew.-%, vorzugsweise mindestens 90 Gew.-%, insbesondere wenigstens 95 Gew.-%, bezogen auf das Gesamtgewicht des Copolymers, in gefällter Form vorliegt.

Die Halbwertszeit eines Initiators kann nach üblichen, dem Fachmann bekannten Verfahren bestimmt werden, wie z. B. in der Druckschrift "Initiators for high polymers", Akzo Nobel, Nr. 10737, beschrieben. Vorzugsweise liegt die Halbwertszeit des ersten Polymerisationsinitiators bei der ersten Polymerisationstemperatur und des zweiten Polymerisationsinitiators bei der zweiten Polymerisationstemperatur in einem Bereich von etwa 1 Minute bis 3 Stunden, besonders bevorzugt 5 Minuten bis 2,5 Stunden. Gewünschtenfalls können auch kürzere Halbwertszeiten z. B. von 1 Sekunde bis 1 Minute oder längere Halbwertszeiten als 3 Stunden zum Einsatz kommen, solange sichergestellt ist, dass der/die bei der höheren Temperatur zerfallende(n) Initiator(en) im Wesentlichen während der zweiten Polymerisationsphase in Radikale zerfällt.

Zusätzlich zu der ersten und zweiten Polymerisationsphase können weitere Polymerisationsphasen bei davon verschiedenen Polymerisationstemperaturen angewandt werden. So ist es z. B. möglich, eine erste Polymerisationsphase bei einer ersten Polymerisationstemperatur durchzuführen, die so gewählt ist, dass eine kontrollierte Polymerisation (d. h. z. B. unter Vermeidung eines unerwünschten Temperaturanstiegs durch die Reaktionswärme, einer zu hohen Reaktionsgeschwindigkeit, etc.) erfolgt. Anschließend kann sich z. B. eine Nachpolymerisation bei einer Temperatur anschließen, die oberhalb der ersten und unterhalb der zweiten Polymerisationstemperatur liegt und die so gewählt ist, dass der/die bei der höheren Temperatur zerfallende(n) Initiator(en) im Wesentlichen nicht in Radikale zerfallen. Nach Abschluss dieser Nachpolymerisation, zu der gewünschtenfalls nochmals der bei der niedrigeren Temperatur zerfallende Initiator und/oder ein anderer unter den Bedingungen der Nachpolymerisation zerfallender Initiator zugegeben werden kann, kann sich dann die zweite Polymerisationsphase anschließen.

Vorzugsweise enthält das eingesetzte Initiatorsystem wenigstens zwei Initiatoren, deren Zerfallstemperaturen sich um wenigstens 15°C voneinander unterscheiden.
Der bei der niedrigeren Temperatur zerfallende Initiator weist vorzugsweise eine Zerfallstemperatur von 50 bis 100 °C auf.
Der bei der höheren Temperatur zerfallende Initiator weist vorzugsweise eine Zerfallstemperatur von 80 bis 150 °C auf.

Vorzugsweise wird der bei der höheren Temperatur zerfallende Initiator zu Beginn der Copolymerisation vorgelegt oder vor oder während der Fällung des Copolymers zugegeben.

Vorzugsweise wird der bei der höheren Temperatur zerfallende Initiator zu Beginn der Copolymerisation vorgelegt oder vor der Fällung des Copolymers zugegeben.

Bei einer bevorzugten Initiatorkombination handelt es sich bei dem bei der niedrigeren Temperatur zerfallenden Initiator um Trigonox^{®} EHP (Bis(2-ethylhexyl)peroxydicarbo-nat, CAS-Nr. 16111-62-9) und ist der bei der höheren Temperatur zerfallende Initiator ausgewählt unter tert.-Butylperoxypivalat (z. B. Luperox^{®} 11 M75 der Fa. Atochem), tert.-Butylperoctoat, Lauroylperoxid (LPO, CAS-Nr. 105-74-8) oder 2,5-Dimethyl-2,5-bis(t-butylperoxy)hexan (Trigonox^{®} 101).

Eine weitere bevorzugte Initiatorkombination enthält Trigonox^{®} EHP oder 2,2'-Azobis(2.4-dimethylvaleronitrile) (Wako^{®} V65) und tert.-Butylperoctoat.

Eine weitere bevorzugte Initiatorkombination enthält Lauroylperoxid und tert.-Butylperoctoat oder 2,5-Dimethyl-2,5-bis(t-butylperoxy)hexan (Trigonox^{®} 101).

Eine weitere bevorzugte Initiatorkombination enthält tert.-Butylperoxypivalat (Luperox 11 M75 und tert.-Butylperoctoat oder 2,5-Dimethyl-2,5-bis(t-butylperoxy)hexan (Trigo-nox^{®} 101).

Eine weitere bevorzugte Initiatorkombination enthält tert.-Butylperoctoat und 2,5-Dimethyl-2,5-bis(t-butylperoxy)hexan (Trigonox^{®} 101).

### Vernetzer e)

Die erfindungsgemäßen Fällungspolymerisate können gewünschtenfalls wenigstens einen Vernetzer e), d.h. eine Verbindung mit zwei oder mehr als zwei ethylenisch ungesättigten, nichtkonjugierten Doppelbindungen einpolymerisiert enthalten. Vorzugsweise werden Vernetzer in einer Menge von 0,01 bis 1 Gew.-%, besonders bevorzugt 0,1 bis 0,5 Gew.-%, bezogen auf das Gesamtgewicht der zur Polymerisation eingesetzten Monomere, verwendet.

Geeignete Vernetzer sind beispielsweise die in WO 2007/010035, S.17, Z.20 bis S.19, Z.18 genannten, worauf hiermit in vollem Umfang Bezug genommen wird.

Ganz besonders bevorzugt als Vernetzer e) sind Ethylenglykoldi(meth)acrylat, Poly-ethylenglykoldi(meth)acrylate, Pentaerythrittriallylether, Methylenbisacrylamid, N,N'-Divinylethylenharnstoff, Triallylamin und Triallylmonoalkylammoniumsalze.
Am meisten bevorzugt als Vernetzer e) ist Pentaerythrittriallylether.

Im Rahmen dieser Erfindung bezeichnet man die Gesamtheit aller Substanzen, die sich bereits vor dem Einsetzen der Polymerisation im Reaktionsgefäß befindet, als Vorlage. Diese Vorlage umfasst bevorzugt wenigstens ein Lösungsmittel. Allerdings kann die Vorlage zusätzlich zum Lösungsmittel auch bereits ein oder mehrere Monomere umfassen. Die Vorlage kann auch weitere Substanzen umfassen wie Initiator, Vernetzer oder Regler.

In einer Ausführungsform der vorliegenden Erfindung umfasst die Vorlage Lösungsmittel, einen Teil der Monomere sowie einen Teil des Initiators.

In einer weiteren Ausführungsform der Erfindung umfasst die Vorlage im Bereich von 10 bis 30 Gew.-%, bevorzugt im Bereich von 15 bis 25 Gew.% der Gesamtmenge aller einzupolymerisierenden Monomere.

In einer weiteren Ausführungsform der Erfindung umfasst die Vorlage im Bereich von 10 bis 20 Gew.-%, bevorzugt im Bereich von 13 bis 17 Gew.% der Gesamtmenge des einzusetzenden Initiators.

In einer weiteren Ausführungsform der Erfindung umfasst die Vorlage Lösungsmittel und einen Teil der Monomere aber keinen Initiator.

In einer weiteren Ausführungsform der Erfindung umfasst die Vorlage zwar Lösungsmittel aber weder Monomere noch Initiator.

In einer Ausführungsform der Erfindung liegt die Menge an von Lösungsmitteln verschiedenen Substanzen in der Reaktionsmischung im Bereich von 10 bis 30 Gew.-%, bevorzugt im Bereich von 15 bis 25 Gew.-%, besonders bevorzugt im Bereich von 18 bis 22 Gew.-%, jeweils bezogen auf das Gesamtgewicht der Reaktionsmischung. Der Anteil der von Lösungsmitteln verschiedenen Substanzen in der Reaktionsmischung wird auch als Feststoffgehalt (kurz "FG") bezeichnet.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Die Temperatur bei der Haupt- und der Nachpolymerisation beträgt vorzugsweise höchstens 100°C (Hauptreaktion) und 130°C (Nachpolymerisation).

Das ausgefallene Polymer wird nach der Polymerisation bzw. nach dem Nachpolymerisationsschritt aus der Reaktionsmischung abgetrennt.
Zu dieser Abtrennung kann jede übliche Methode zur Abtrennung von Polymeren bei der konventionellen Fällungspolymerisation verwendet werden.
Geeignete Methoden zur Abtrennung der Fällungspolymerisate von den übrigen Bestandteilen der Reaktionsmischung sind beispielsweise Filtration, Zentrifugation, Eindampfen des Lösungsmittels oder Kombinationen dieser Methoden.
Zur weiteren Reinigung der Fällungspolymerisate wird bevorzugt eine Wäsche der Polymerisate durchgeführt. Dafür werden bevorzugt die gleichen oder ähnliche Lösungsmittel verwendet, wie sie auch für die vorausgegangene Fällungspolymerisation verwendet worden sind.
Das Waschen der Fällungspolymerisate ist ein dem Fachmann bekannter Vorgang.

In einer bevorzugten Ausführungsform der Erfindung werden die Polymere getrocknet. Diese Trocknung kann auf verschiedene Art und Weise erfolgen.
Die dem Fachmann bekannten Trocknungsmethoden wie beispielsweise Erwärmung, Lagern unter vermindertem Druck, Lagern unter üblichen Standardbedingungen und Kombinationen dieser Methoden sind dafür geeignet.

Die pulverförmigen erfindungsgemäßen Fällungspolymerisate haben im Vergleich zu Lösungspolymerisaten den Vorteil der besseren Lagerfähigkeit und einer einfacheren Transportmöglichkeit und zeigen in der Regel eine geringere Neigung für Keimbefall. Die vorzugsweise erhaltenen Polymer-Trockenpulver lassen sich vorteilhafterweise durch Lösen bzw. Dispergieren in Wasser in eine wässrige Polymer-Lösung bzw. Polymer-Dispersion überführen.

Enthalten die erfindungsgemäßen Fällungspolymerisate Säuregruppen, so können diese mit einer Base teilweise oder vollständig neutralisiert werden. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Trüsopropylamin. Bevorzugt sind Aminoalkohole, z.B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Besonders für den Einsatz in Haarbehandlungsmitteln haben sich zur Neutralisation der Säuregruppen enthaltenden Polymere 2-Amino-2-methyl-1-propanol (abgekürzt als "AMP"), 2-Amino-2-ethylpropan-1,3-diol, Diethylaminopropylamin und Triisopropanolamin bewährt. Ebenfalls geeignet sind die in WO 03/099253, S.2, Zeile 20 bis S.3, Zeile 6 genannten Basen, worauf hiermit Bezug genommen wird. Die Neutralisation der Säuregruppen kann auch mit Hilfe von Mischungen mehrerer Basen vorgenommen werden, z. B. Mischungen aus Natronlauge und Triisopropanolamin. Die Neutralisation kann je nach Anwendungszweck partiell oder vollständig erfolgen.

Enthalten die erfindungsgemäßen Fällungspolymerisate kationogene Gruppen wie beispielsweise Aminogruppen, so können aus diesen Aminogruppen durch Protonierung, z. B. mit ein- oder mehrwertigen Carbonsäuren wie Milchsäure oder Weinsäure oder mit Mineralsäuren wie Phosphorsäure, Schwefelsäure und Salzsäure oder durch Quaternisierung, z. B. mit Alkylierungsmitteln, wie C₁- bis C₄-Alkylhalogeniden oder - sulfaten, kationische Gruppen erzeugt werden.
Beispiele für geeignete Alkylierungsmittel sind Ethylchlorid, Ethylbromid, Methylchlorid, Methylbromid, Dimethylsulfat und Diethylsulfat.

Sollen die erfindungsgemäßen Fällungspolymerisate sowohl quaternisiert als auch neutralisiert werden, so erfolgt vorzugsweise zuerst die Quaternisierung und anschließend die Neutralisierung.

Eine Ausführungsform der Erfindung sind erfindungsgemäße Fällungspolymerisate, wobei Monomer a) N-Vinylpyrrolidon (im Rahmen dieser Erfindung auch kurz als "VP" bezeichnet) ist oder umfasst.

Eine Ausführungsform der Erfindung sind Fällungspolymerisate, die als wenigstens ein Monomer a) N-Vinylpyrrolidon (im Rahmen dieser Erfindung auch kurz als "VP" bezeichnet) und als wenigstens ein Monomer c1) (Meth)acrylsäure einpolymerisiert enthalten.

Eine Ausführungsform der Erfindung sind Fällungspolymerisate, die als ein Monomer a) N-Vinylpyrrolidon und als ein Monomer c2) N-Vinylimidazol (im Rahmen dieser Erfindung auch kurz als "VI" bezeichnet) einpolymerisiert enthalten.

Eine Ausführungsform der Erfindung sind Fällungspolymerisate, die als ein Monomer a) N-Vinylpyrrolidon und als ein Monomer c3) DMAEMA einpolymerisiert enthalten.

Eine Ausführungsform der Erfindung sind Fällungspolymerisate, die als ein Monomer a) N-Vinylpyrrolidon und als ein Monomer c4) DMAPMAM einpolymerisiert enthalten.

Eine Ausführungsform der Erfindung sind Fällungspolymerisate, die als ein Monomer a) N-Vinylpyrrolidon, als ein erstes Monomer c) (Meth)acrylsäure und als ein zweites Monomer c) eines oder mehrere aus VI, DMAPMAM und DMAEMA einpolymerisiert enthalten. Bevorzugt ist dabei ein Gewichtsverhältnis von anionischem/anionigenem Monomer zu kationischem/kationogenen Monomer kleiner oder gleich 1:2 oder größer oder gleich 2:1.

Eine weitere Ausführungsform der Erfindung sind Fällungspolymerisate, die
a) 90 bis 99,5 Gew.-% N-Vinylpyrrolidon,
b) 0,5 bis 10 Gew.-% Methacrylamid (MAM)
c) c1) 0 bis 10 Gew.-% (Meth)acrylsäure und
c2) 0 bis 10 Gew.-% N-Vinylimidazol einpolymerisiert enthalten, wobei die Gesamtmenge aller einpolymerisierten Monomere 100 Gew.-% beträgt.

Eine weitere Ausführungsform der Erfindung sind Fällungspolymerisate, die
a) 85 bis 97 Gew.-% N-Vinylpyrrolidon,
b) 0,5 bis 5 Gew.-% Methacrylamid,
c) c2) 1,5 bis 10 Gew.-% N-Vinylimidazol einpolymerisiert enthalten, wobei die Gesamtmenge aller einpolymerisierten Monomere 100 Gew.-% beträgt.

Eine weitere Ausführungsform der Erfindung sind Fällungspolymerisate, die
a) 85 bis 97 Gew.-% N-Vinylpyrrolidon,
b) 0,5 bis 5 Gew.-% Methacrylamid,
c) c1) 1,5 bis 10 Gew.-% Acrylsäure einpolymerisiert enthalten, wobei die Gesamtmenge aller einpolymerisierten Monomere 100 Gew.-% beträgt.

Eine weitere Ausführungsform der Erfindung sind Fällungspolymerisate, die
a) 80 bis 95 Gew.-% N-Vinylpyrrolidon,
b) 0,2 bis 5 Gew.-% Ureidomethacrylat (UMA)
c) c1) 1,5 bis 10 Gew.-% (Meth)acrylsäure und
   c8-1) 0 bis 3 Gew.-% C₁₆-C₁₈-Alkyl-PEG₁₁₀₀- Methacrylat einpolymerisiert enthalten,
   wobei die Gesamtmenge aller einpolymerisierten Monomere 100 Gew.-% beträgt.

Eine weitere Ausführungsform der Erfindung sind Fällungspolymerisate, die
a) 80 bis 95 Gew.-% N-Vinylpyrrolidon,
b) 0,2 bis 5 Gew.-% Ureidomethacrylat (UMA)
c) c1) 1,5 bis 10 Gew.-% N-Vinylimidazol (VI)
   c8-1) 0 bis 3 Gew.-% C₁₆-C₁₈-Alkyl-PEG₁₁₀₀-MA und
d) 0 bis 10 Gew.-% Methylmethacrylat (c7) und/oder Methacrylsäure (c1) einpolymerisiert enthalten, wobei die Gesamtmenge aller einpolymerisierten Monomere 100 Gew.-% beträgt.

In einer weiteren Ausführungsform der Erfindung haben die vorgenannten Fällungspolymerisate im Bereich von 0,01 bis 1 Gew.-%, bevorzugt im Bereich von 0,1 bis 0,5 Gew.-% eines Vernetzers, bevorzugt Pentaerythrittriallylether (PETAE), einpolymerisiert, wobei die Gesamtmenge aller einpolymerisierten Monomere 100 Gew.-% beträgt.

In einer weiteren Ausführungsform der Erfindung haben die vorgenannten Fällungspolymerisate als Monomer b) b1) Methacrylamid und b3) Allylgluconamid einpolymerisiert.

In einer weiteren Ausführungsform der Erfindung haben die vorgenannten Fällungspolymerisate als Monomer b) b3) Allylgluconamid anstelle von b1) Methacrylamid einpolymerisiert.

In einer weiteren Ausführungsform der Erfindung haben die vorgenannten Fällungspolymerisate als Monomer b) b2) Ureidomethacrylat einpolymerisiert.

Eine weitere Ausführungsform der Erfindung sind nichtionische Fällungspolymere enthaltend 95-99 Gew.-% N-Vinylpyrrolidon und 1-5 Gew.-% Methacrylamid (MAM) einpolymerisiert, wobei die Gesamtmenge aller einpolymerisierten Monomere 100 Gew.-% beträgt, wie beispielsweise:

| Fällungspolymer | VP | MAM | | |
|---|---|---|---|---|
| N1 | 98 | 2 | | |
| N2 | 95 | 5 | | |

Eine weitere Ausführungsform der Erfindung sind kationische/kationogene Fällungspolymere enthaltend 88-92 Gew.-% N-Vinylpyrrolidon (VP), 3-6 Gew.-% Methacrylamid (MAM), 4-8 Gew.-% eines kationischen/kationogenen Monomers c), bevorzugt N-Vinylimidazol (VI) und 0-2 Gew.-% eines Vernetzers, bevorzugt Pentaerythrittriallylether (PETAE), einpolymerisiert, wobei die Gesamtmenge aller einpolymerisierten Monomere 100 Gew.-% beträgt, wie beispielsweise:

| Fällungspolymer | VP | MAM | VI | PETAE |
|---|---|---|---|---|
| K1 | 90 | 3 | 7 | |
| K2 | 90 | 5 | 5 | |
| K3 | 90 | 3 | 6,8 | 0,2 |

Eine weitere Ausführungsform der Erfindung sind kationische/kationogene Fällungspolymere enthaltend 80-90 Gew.-% N-Vinylpyrrolidon (VP), 0-15 Gew.-% Methacrylamid (MAM), 0,5-3 Gew.-% Ureidomethacrylat (UMA), 0-9 Gew.-% Methylmethacrylat (MMA), 4-8 Gew.-% eines kationischen/kationogenen Monomers c), insbesondere N-Vinylimidazol (VI), 0 bis 3 Gew.-% Polyether(meth)acrylat und 0-2 Gew.-% eines Vernetzers einpolymerisiert, wobei die Gesamtmenge aller einpolymerisierten Monomere 100 Gew.-% beträgt, wie beispielsweise:

| Fällungspolymer | VP | MAM | Plex^{®}6844-O | VI | Plex^{®}6877-O |
|---|---|---|---|---|---|
| K4 | 80 | 10 | 6 | 4 | -- |
| K5 | 88 | -- | 4 | 6 | 2 |

Eine weitere Ausführungsform der Erfindung sind kationische/kationogene Fällungspolymere enthaltend 80-90 Gew.-% N-Vinylpyrrolidon (VP), 0,1-3 Gew.-% Allyl-D-Gluconamid (AGA), 10-18 Gew.-% eines kationischen/kationogenen Monomers c), insbesondere N-Vinylimidazol (VI), 0-6 Gew.-% Methylmethacrylat, 0,5 bis 3 Gew.-% Polyether(meth)acrylat und 0-2 Gew.-% eines Vernetzers einpolymerisiert, wobei die Gesamtmenge aller einpolymerisierten Monomere 100 Gew.-% beträgt, wie beispielsweise:

| Fällungspolymer | VP | AGA | VI | Plex^{®}6877-O |
|---|---|---|---|---|
| K6 | 80 | 1 | 15 | 4 |

Eine weitere Ausführungsform der Erfindung sind anionische/anionogene Fällungspolymere enthaltend 85-97 Gew.-% N-Vinylpyrrolidon (VP), 1-4 Gew.-% Methacrylamid (MAM), 1-6 Gew.-% Acrylsäure (AS) und 0-2 Gew.-% eines Vernetzers, bevorzugt Pentaerythrittriallylether (PETAE), einpolymerisiert, wobei die Gesamtmenge aller einpolymerisierten Monomere 100 Gew.-% beträgt, wie beispielsweise

| Fällungspolymer | VP | MAM | AS | |
|---|---|---|---|---|
| A1 | 95 | 3 | 2 | |
| A2 | 93 | 2 | 5 | |

Eine weitere Ausführungsform der Erfindung sind anionische/anionogene Fällungspolymere enthaltend 85-95 Gew.-% N-Vinylpyrrolidon (VP), 0,1-2 Gew.-% Ureidomethacrylat, 0-6 Gew.-% Methylmethacrylat, 5-10 Gew.-% Acrylsäure (AS), 0-3 Gew.-% Polyether(meth)acrylat, 0-9 Gew.-% Methylmethacrylat und 0-2 Gew.-% eines Vernetzers einpolymerisiert, wobei die Gesamtmenge aller einpolymerisierten Monomere 100 Gew.-% beträgt, wie beispielsweise

| Fällungspolymer | VP | Plex^{®}6844-O | AS | Plex^{®}6877-O |
|---|---|---|---|---|
| A3 | 92 | 2 | 6 | |
| A4 | 86 | 4 | 10 | |
| A5 | 80 | 4 | 10 | 6 |

Eine weitere Ausführungsform der Erfindung sind anionische/anionogene Fällungspolymere enthaltend 85-95 Gew.-% N-Vinylpyrrolidon (VP), 0,1-2 Gew.-% Allyl-D-Gluconamid, 8-12 Gew.-% Acrylsäure (AS) und 0-2 Gew.-% eines Vernetzers einpolymerisiert, wobei die Gesamtmenge aller einpolymerisierten Monomere 100 Gew.-% beträgt, wie beispielsweise

| Fällungspolymer | VP | AGA | AS | |
|---|---|---|---|---|
| A6 | 90 | 1 | 9 | |

Die erfindungsgemäßen Fällungspolymerisate mit hohen Gehalten an einpolymerisiertem N-Vinylpyrrolidon besitzen besonders vorteilhafte Eigenschaften. Diese sind beispielsweise das höhere Molekulargewicht, die geringere Klebrigkeit, die erhöhte Verdickungswirkung und die bessere Befähigung zur Bildung pulverförmiger Formulierungen im Vergleich zu Homopolymeren des N-Vinylpyrrolidon oder Polymeren mit vergleichbaren Gehalten an einpolymerisiertem N-Vinylpyrrolidon, die nach anderen Polymerisationsverfahren wie beispielsweise der Lösungspolymerisation erhalten werden.

### Verwendung der erfindungsgemäßen Fällungspolymerisate

Die Erfindung betrifft weiterhin kosmetische Zusammensetzungen ausgewählt aus Gelcremes, Hydroformulierungen, Stiftformulierungen, kosmetischen Ölen und Ölgelen, Mascara, Selbstbräunern, Gesichtspflegemitteln, Körperpflegemitteln, After-Sun-Präparaten, Haarverformungsmitteln und Haarfestigern, die die erfindungsgemäßen Fällungspolymerisate enthalten.

Weitere erfindungsgemäße kosmetische Zusammensetzungen sind hautkosmetische Zusammensetzungen, insbesondere solche zur Pflege der Haut, die die erfindungsgemäßen Fällungspolymerisate enthalten. Diese liegen insbesondere als W/O- oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.

Weiterhin eignen sich die erfindungsgemäßen Fällungspolymerisate als Inhaltsstoffe für hautkosmetische Zubereitungen wie Gesichtswässer, Gesichtsmasken, Deodorantien und andere kosmetische Lotionen und für die Verwendung in der dekorativen Kosmetik, beispielsweise als Abdeckstift, Theaterfarbe, in Mascara und Lidschatten, Lippenstiften, Kajalstiften, Eyelinern, Makeup, Grundierungen, Rouges und Pudern und Augenbrauenstiften.

Außerdem können die erfindungsgemäßen Fällungspolymerisate verwendet werden in Nose-Strips zur Porenreinigung, in Antiaknemitteln, Repellentien, Rasiermitteln, Haarentfernungsmitteln, Intimpflegemitteln, Fußpflegemitteln sowie in der Babypflege.

Weitere bevorzugte erfindungsgemäße Zubereitungen sind Wasch-, Dusch- und Bade-präparate, die die erfindungsgemäßen Fällungspolymerisate enthalten.

Unter Wasch-, Dusch- und Badepräparaten werden im Rahmen dieser Erfindung Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes verstanden.

Die erfindungsgemäßen kosmetischen Zusammensetzungen können als wässrige oder wässrig-alkoholische Lösungen, O/W- sowie W/O-Emulsionen, Hydrodispersionsformulierungen, feststoffstablilisierte Formulierungen, Stiftformulierungen, PIT-Formulierungen, in Form von Cremes, Schäumen, Sprays (Pumpspray oder Aerosol), Gelen, Gelsprays, Lotionen, Ölen, Ölgelen oder Mousse vorliegen und dementsprechend mit üblichen weiteren Hilfsstoffen formuliert werden.

Die erfindungsgemäßen kosmetischen Zubereitungen enthalten bevorzugt wenigstens ein erfindungsgemäßes Fällungspolymerisat, wenigstens einen kosmetisch akzeptablen Träger und wenigstens einen davon verschiedenen Bestandteil der ausgewählt ist unter kosmetisch aktiven Wirkstoffen, Emulgatoren, Tensiden, Konservierungsmitteln, Parfümölen, weiteren Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Erfindungsgemäß bevorzugte Haarpflegemittel sind ausgewählt aus Vorbehandlungsmitteln, Haarspülungen, Haarconditionern, Haarbalsamen, leave-on-Haarkuren, rinseoff-Haarkuren, Haarwässern, Pomaden, Frisiercremes, Frisierlotionen, Frisiergelen, Spitzenfluids, Hot-Oil-Treatments und Schaumkuren.

Bevorzugt werden die erfindungsgemäßen Fällungspolymerisate als rheologiemodifizierende Filmbildner, Haarfestiger und Konditioniermittel zur Herstellung von kosmetischen, bevorzugt haarkosmetischen Zubereitungen verwendet.

Ein weiterer Gegenstand der Erfindung sind also kosmetische, insbesondere haarkosmetische Zubereitungen enthaltend die erfindungsgemäßen Fällungspolymerisate.

Bevorzugte haarkosmetische Zusammensetzungen sind Haarreinigungsmittel, Shampoos, Haarpflegemittel und Haarfestiger, darunter insbesondere Haarfestigergele.

Die erfindungsgemäßen Fällungspolymerisate wirken insbesondere als filmbildende und/oder konditionierende Rheologiemodifizierungsmittel. Sie eignen sich somit speziell für Haarfestiger als "verdickende Festiger" oder "festigende Verdicker" und in Haarpflegemitteln als "konditionierende Verdicker".

Prinzipiell können die erfindungsgemäßen Fällungspolymerisate bei ihrer Verwendung in mehrphasigen Zusammensetzungen wie beispielsweise O/W und W/O, sowohl in der Wasserphase als auch in der Ölphase eingesetzt werden. Im Allgemeinen enthalten heterogenphasige flüssig/flüssig-Zusammensetzungen die erfindungsgemäßen Fällungspolymerisate im Wesentlichen in der Wasserphase.

Ein weiterer Gegenstand der Erfindung sind haarkosmetische Mittel enthaltend
A) wenigstens ein erfindungsgemäßes Fällungspolymerisat,
B) gegebenenfalls wenigstens ein von A) verschiedenes Haarpolymer,
C) wenigstens einen kosmetisch akzeptablen Träger, und
D) gegebenenfalls wenigstens einen von A) und B) verschiedenen kosmetisch akzeptablen Wirk- und/oder Hilfsstoff.

Die erfindungsgemäßen Fällungspolymerisate zeichnen sich vorteilhafterweise nicht nur durch filmbildende, sondern auch durch rheologiemodifizierende Eigenschaften aus. Sie können somit in den haarkosmetischen Mitteln auch als haarfestigende Komponente eingesetzt werden, so dass der Einsatz weiterer Festigerpolymere nur in verringerter Menge erforderlich ist oder sogar ganz überflüssig sein kann.
Die erfindungsgemäßen Fällungspolymerisate zeichnen sich vorteilhafterweise auch durch konditionierende Eigenschaften aus und können die sensorischen Eigenschaften des Haars verbessern, z. B. ihm Geschmeidigkeit und Glanz verleihen.

Die haarkosmetischen Mittel enthalten die erfindungsgemäßen Fällungspolymerisate vorzugsweise in einem Anteil von etwa 0,1 bis 10 Gew.-%, besonders bevorzugt 0,2 bis 6 Gew.-%, insbesondere 0,3 bis 3 Gew.-%, bezogen auf das Gesamtgewicht des Mittels.

Beispiele für geeignete Haarpolymere B) und deren bevorzugte Mengen sind in WO 2007/010035, S.68, Z.32 bis S.70, Z.22 ausführlich beschrieben. Auf diese Textstelle wird hiermit in vollem Umfang Bezug genommen.

Vorzugsweise weisen die Zusammensetzungen eine Trägerkomponente C) auf, die ausgewählt ist unter Wasser, hydrophilen Komponenten, hydrophoben Komponenten und Mischungen davon.

Geeignete Trägerkomponenten C) sind in WO 2007/010035, S.70, Z.28 bis S.71, Z.37 ausführlich beschrieben. Auf diese Textstelle wird hiermit in vollem Umfang Bezug genommen.

Zusätzlich können die erfindungsgemäßen Mittel als Komponente D) wenigstens einen weiteren, von A) und B) verschiedenen kosmetischen Wirk- oder Hilfsstoff enthalten. Geeignete Komponenten D) sind in WO 2007/010035, S.72, Z.2 bis S.72, Z.13 ausführlich beschrieben. Auf diese Textstelle wird hiermit in vollem Umfang Bezug genommen.

Die erfindungsgemäßen Fällungspolymerisate können gemeinsam mit bekannten Verdickern verwendet werden. Geeignete Verdicker sind in WO 2007/010035, S.72, Z.15 bis S.72, Z.24 ausführlich beschrieben. Auf diese Textstelle wird hiermit in vollem Umfang Bezug genommen.

### Konditionierungsmittel

Als Konditionierungsmittel für die erfindungsgemäßen kosmetischen Zubereitungen werden bevorzugt diejenigen Konditionierungsmittel gewählt, die auf Seite 34, Zeile 24 bis Seite 37, Zeile 10 der WO 2006/106140 beschrieben sind, worauf hiermit Bezug genommen wird.

### Verdicker

Für Gele, Shampoos und Haarpflegemittel geeignete Verdicker sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird. Geeignete weitere Verdickungsmittel für die erfindungsgemäßen kosmetischen Zubereitungen sind beispielsweise auch auf Seite 37, Zeile 12 bis Seite 38, Zeile 8 der WO 2006/106140 beschrieben, worauf hiermit Bezug genommen wird.

### Konservierungsmittel

Geeignete Konservierungsmittel für die erfindungsgemäßen kosmetischen Zusammensetzungen sind beispielsweise auf Seite 38, Zeile 10 bis Seite 39, Zeile 18 der WO 2006/106140 beschrieben, worauf hiermit Bezug genommen wird.

### UV- Lichtschutzfilter

Geeignete UV- Lichtschutzfilter für die erfindungsgemäßen kosmetischen Zusammensetzungen sind beispielsweise auf Seite 39, Zeile 20 bis Seite 41 Zeile 10 der WO 2006/106140 beschrieben, worauf hiermit Bezug genommen wird.

### Antioxidantien

Geeignete Antioxidantien für die erfindungsgemäßen kosmetischen Zusammensetzungen sind beispielsweise auf Seite 41, Zeile 12 bis Seite 42 Zeile 33 der WO 2006/106140 beschrieben, worauf hiermit Bezug genommen wird.

### Dispergiermittel

Wenn in den erfindungsgemäßen Zusammensetzungen unlösliche Wirkstoffe, z.B. Antischuppenwirkstoffe oder Siliconöle, dispergiert und auf Dauer in Schwebe gehalten werden sollen, werden bevorzugt Dispergiermittel und Verdicker wie z. B. Magnesium-Aluminium-Silicate, Bentonite, Fettacyl-Derivate, Polyvinylpyrrolidon oder Hydrokolloide, z. B. Xanthan Gum oder Carbomere, eingesetzt.

Die Zusammensetzungen können weitere in der Kosmetik übliche Zusatzstoffe, beispielsweise Parfüm, Farbstoffe, rückfettende Agentien, Komplexierungs- und Sequestrierungsagentien, Perlglanzagentien, Pflanzenextrakte, Vitamine, Wirkstoffe, Pigmente, die eine färbende Wirkung haben, weichmachende, anfeuchtende und/oder feuchthaltende Substanzen, oder andere übliche Bestandteile wie Alkohole, Polyole, Polymere, organische Säuren zur pH-Wert-Einstellung, Schaumstabilisatoren, Elektrolyte, organische Lösemittel oder Silikonderivate enthalten.
Hinsichtlich der genannten, dem Fachmann bekannten weiteren Inhaltsstoffe für die Zusammensetzungen sei auf "Kosmetik und Hygiene von Kopf bis Fuß", Hrsg. W. Umbach, 3. Auflage, Wiley-VCH, 2004, S.123-128 verwiesen, worauf hiermit Bezug genommen wird.

Die erfindungsgemäßen Zusammensetzungen wie Haarsprays, Gele, Shampoos und Haarpflegemittel enthalten gegebenenfalls ethoxylierte Öle ausgewählt aus der Gruppe der ethoxylierten Glycerin-Fettsäureester, insbesondere bevorzugt PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fett-Säuren, PEG-26 Jojobaalkohol), Glycereth-5 Cocoat, PEG-9 Kokosfettsäureglyceride, PEG-7 Glycerylcocoat, PEG-45 Palmkernölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkernölglycerid-PEG-6 Ester, PEG-20 Maisölglyceride, PEG- 18 Glyceryloleat-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat.
Bevorzugte ethoxylierte Öle sind PEG-7 Glycerylcocoat, PEG-9 Kokosglyceride, PEG-40 Hydriertes Rizinusöl, PEG-200 hydriertes Glycerylpalmat.
Ethoxylierte Glycerin-Fettsäureester werden in wässrigen Reinigungsrezepturen zu verschiedenen Zwecken eingesetzt. Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt.

### Wirkstoffe

Vorteilhafte Wirkstoffe für die erfindungsgemäßen kosmetischen Zusammensetzungen sind beispielsweise auf Seite 44, Zeile 24 bis Seite 49 Zeile 39 der WO 2006/106140 beschrieben, worauf hiermit Bezug genommen wird.

### UV-Lichtschutzmittel

Die erfindungsgemäßen Zusammensetzungen enthalten in einer bevorzugten Ausführungsform UV-Lichtschutzmittel zum Schutz der Haut und/oder der Haare. Geeignete UV-Lichtschutzmittel sind in der WO 2006/106114, S.24, Z.4 bis S.27, Z.27 ausführlich beschrieben, worauf hiermit in vollem Umfang Bezug genommen wird.

### Perlglanzwachse

Geeignete Perlglanzwachse für die erfindungsgemäßen kosmetischen Zusammensetzungen sind beispielsweise auf Seite 50, Zeile 1 bis Zeile 16 der WO 2006/106140 beschrieben, worauf hiermit in vollem Umfang Bezug genommen wird.

### Emulgatoren

Die erfindungsgemäßen kosmetischen Zusammensetzungen liegen in einer bevorzugten Ausführungsform der Erfindung in Form von Emulsionen vor. Die Herstellung solcher Emulsionen erfolgt nach bekannten Methoden. Geeignete Emulgatoren für die erfindungsgemäßen Emulsionen sind beispielsweise auf Seite 50, Zeile 18 bis Seite 53, Zeile 4 der WO 2006/106140 beschrieben, worauf hiermit in vollem Umfang Bezug genommen wird.

### Parfümöle

Sollen den erfindungsgemäßen kosmetischen Zusammensetzungen Parfumöle zugesetzt werden, so sind geeignete Parfümöle beispielsweise auf Seite 53, Zeile 10 bis Seite 54, Zeile 3 der WO 2006/106140 beschrieben, worauf hiermit in vollem Umfang Bezug genommen wird.

### Pigmente

Gegebenenfalls enthalten die erfindungsgemäßen kosmetischen Zusammensetzungen weiterhin Pigmente. Geeignete Pigmente für die erfindungsgemäßen Zusammensetzungen sind beispielsweise auf Seite 54, Zeile 5 bis Seite 55, Zeile 19 der WO 2006/106140 beschrieben, worauf hiermit in vollem Umfang Bezug genommen wird.

### Nanopartikel

Gegebenenfalls enthalten die erfindungsgemäßen Zusammensetzungen wasserunlösliche Nanopartikel, also Teilchen mit einer Teilchengröße im Bereich von 1 bis 200, bevorzugt von 5 bis 100 nm. Bevorzugte Nanopartikel sind Nanopartikel von Metalloxiden, insbesondere von Zinkoxid und/oder Titandioxid.

### Polymere

Die erfindungsgemäßen kosmetischen Zusammensetzungen enthalten in einer bevorzugten Ausführungsform außer den erfindungsgemäßen Fällungspolymerisaten noch weitere Polymere. Geeignete weitere Polymere sind beispielsweise auf Seite 55, Zeile 21 bis Seite 63, Zeile 2 der WO 2006/106140 beschrieben. Auf den Inhalt der genannten Textstelle wird hiermit in vollem Umfang Bezug genommen.

Die erfindungsgemäßen Fällungspolymerisate sind auch als rheologiemodifizierende Filmbildner in Haargelen, insbesondere sogenannten Stylinggelen geeignet.
Für diesen Zweck besonders geeignet sind diejenigen Fällungspolymerisate, die einen Überschuss an anionischen und/oder anionogenen Gruppen besitzen.
Ein Gegenstand der Erfindung sind also Haargele und Haarfestigergele, die erfindungsgemäße Fällungspolymerisate enthalten, die einen Überschuss an anionischen und/oder anionogenen Gruppen besitzen, d.h. in denen die molare Menge an anionischen und anionogenen Gruppen größer ist als die molare Menge an kationischen und kationogenen Gruppen.

Dies sind insbesondere anionische/anionogene Fällungspolymere, die 85-97 Gew.-% N-Vinylpyrrolidon (VP), 1-4 Gew.-% Methacrylamid (MAM), 1-6 Gew.-% Acrylsäure (AS) und 0-2 Gew.-% eines Vernetzers, bevorzugt Pentaerythrittriallylether (PETAE), einpolymerisiert enthalten, wobei die Gesamtmenge aller einpolymerisierten Monomere 100 Gew.-% beträgt.

Dies sind weiterhin insbesondere anionische/anionogene Fällungspolymere, die 85-95 Gew.-% N-Vinylpyrrolidon (VP), 0,1-2 Gew.-% Ureidomethacrylat, 0-6 Gew.-% Methylmethacrylat, 5-10 Gew.-% Acrylsäure (AS), 0-3 Gew.-% Polyether(meth)acrylat, 0-9 Gew.-% Methylmethacrylat und 0-2 Gew.-% eines Vernetzers einpolymerisiert enthalten, wobei die Gesamtmenge aller einpolymerisierten Monomere 100 Gew.-% beträgt.

Dies sind weiterhin insbesondere anionische/anionogene Fällungspolymere, die 85-95 Gew.-% N-Vinylpyrrolidon (VP), 0,1-2 Gew.-% Allyl-D-Gluconamid (AGA), 8-12 Gew.-% Acrylsäure (AS) und 0-2 Gew.-% eines Vernetzers einpolymerisiert enthalten, wobei die Gesamtmenge aller einpolymerisierten Monomere 100 Gew.-% beträgt.

Eine bevorzugte Ausführungsform der Erfindung sind haarkosmetische Zubereitungen, insbesondere Haarfestiger und Haargele, die neben den erfindungsgemäßen rheologiemodifizierenden Fällungspolymerisaten in der Kosmetik übliche Gelbildner enthalten.
Solche weiteren, üblichen Gelbildner sind leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Eine bevorzugte Ausführungsform der Erfindung sind haarkosmetische Zubereitungen, insbesondere Haarfestiger und Haargele, die wenigstens ein erfindungsgemäßes rheologiemodifizierendes anionisches/anionogenes Fällungspolymerisat und wenigstens einen Verdicker mit der INCI-Bezeichnung Carbomer enthalten. Bevorzugte Verdicker der Bezeichnung Carbomer sind kommerziell beispielsweise unter dem Handelsnamen Carbopol^{®} erhältlich.

Eine weitere bevorzugte Ausführungsform der Erfindung sind haarkosmetische Zubereitungen, insbesondere Haarfestiger und Haargele, die wenigstens ein erfindungsgemäßes Fällungspolymerisat und wenigstens einen anionischen assoziativen Verdicker wie beispielsweise sogenannte HASE-Verdicker (HASE bedeutet "anionic hydrophobically modified alkali-soluble acrylic polymer emulsion") mit den INCI-Bezeichnungen Acrylates/Steareth-20 Methacrylate Copolymer (beispielsweise Aculyne^{®}22), Acrylates/Beheneth-25 Methacrylate Copolymer (beispielsweise Aculyne^{®}28)) oder Acrylates/Steareth-20 Methacrylate Crosspolymer (beispielsweise Aculyne^{®}88) enthalten.

### Haarwaschmittel

Eine bevorzugte Ausführungsform der Erfindung sind Haarwaschmittel und Shampoos enthaltend die erfindungsgemäßen Fällungspolymerisate. Für diese Ausführungsform sind aufgrund ihrer konditionierenden und rheologiemodifizierenden Eigenschaften insbesondere die erfindungsgemäßen kationischen oder kationogenen Fällungspolymerisate geeignet, d.h. diejenigen erfindungsgemäßen Polymerisate, in denen die molare Menge an anionischen und anionogenen Gruppen kleiner ist als die molare Menge an kationischen und kationogenen Gruppen.

Dies sind beispielsweise erfindungsgemäße kationische/kationogene Fällungspolymere, die 88-92 Gew.-% N-Vinylpyrrolidon (VP), 3-6 Gew.-% Methacrylamid (MAM), 4-8 Gew.-% eines kationischen/kationogenen Monomers c), insbesondere N-Vinylimidazol (VI) und 0-2 Gew.-% eines Vernetzers, bevorzugt Pentaerythrittriallylether (PETAE), einpolymerisiert enthalten, wobei die Gesamtmenge aller einpolymerisierten Monomere 100 Gew.-% beträgt.

Dies sind weiterhin erfindungsgemäße kationische/kationogene Fällungspolymere, die 80-90 Gew.-% N-Vinylpyrrolidon (VP), 0-15 Gew.-% Methacrylamid (MAM), 0,5-3 Gew.-% Ureidomethacrylat (UMA), 0-9 Gew.-% Methylmethacrylat (MMA), 4-8 Gew.-% eines kationischen/kationogenen Monomers c), insbesondere N-Vinylimidazol (VI), 0 bis 3 Gew.-% Polyether(meth)acrylat und 0-2 Gew.-% eines Vernetzers einpolymerisiert enthalten, wobei die Gesamtmenge aller einpolymerisierten Monomere 100 Gew.-% beträgt.

Dies sind weiterhin erfindungsgemäße kationische/kationogene Fällungspolymere, die 80-90 Gew.-% N-Vinylpyrrolidon (VP), 0,1-3 Gew.-% Allyl-D-Gluconamid (AGA), 10-18 Gew.-% eines kationischen/kationogenen Monomers c), insbesondere N-Vinylimidazol (VI), 0-6 Gew.-% Methylmethacrylat, 0,5 bis 3 Gew.-% Polyether(meth)acrylat und 0-2 Gew.-% eines Vernetzers einpolymerisiert enthalten, wobei die Gesamtmenge aller einpolymerisierten Monomere 100 Gew.-% beträgt.

An Shampoos und Haarwaschmittel werden je nach Haarqualität oder Kopfhautproblem gegebenenfalls zusätzliche Anforderungen gestellt.
Bevorzugte erfindungsgemäße Shampoos und Haarwaschmittel enthalten anionische Tenside. Weitere bevorzugte erfindungsgemäße Shampoos und Haarwaschmittel enthalten Kombinationen von anionischen und ampholytischen Tensiden. Weitere bevorzugte erfindungsgemäße Shampoos und Haarwaschmittel enthalten Kombinationen von anionischen und zwitterionischen Tensiden. Weitere bevorzugte erfindungsgemäße Shampoos und kosmetische Reinigungsmittel enthalten Kombinationen von anionischen und nichtionischen Tensiden.
Geeignete Tenside aller Typen sind zuvor unter "Tenside" bereits beschrieben. Bevorzugte anionische Tenside sind Alkylsulfate, Alkylpolyglykolethersulfate und E-thercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen. Besonders bevorzugte anionische Tenside sind die Alkali- oder Ammoniumsalze des Laurylethersulfates mit einem Ethoxylierungsgrad von 2 bis 4 EO-Einheiten.
Ein bevorzugtes zwitterionisches Tensid ist das unter der INCI-Bezeichnung Cocamidopropyl Betaine bekannte Fettsäureamid-Derivat.
Besonders bevorzugte ampholytische Tenside sind das N-Kokosalkylaminopropionat, das Kokosacylaminoethylaminopropionat und das N-Lauroylsarcosinat.
Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

### Darreichung

Die erfindungsgemäßen Zubereitungen können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels. Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Dimethylether, Kohlenwasserstoffe (Propan, Butan, Isobutan) geeignet, die allein oder in Mischung, beispielsweise Mischungen aus Dimethylether und Isobutan oder Dimethylether und Butan, miteinander eingesetzt werden können. Auch Druckluft, Stickstoff, Stickstoffdioxid oder Kohlendioxid oder Gemische aus diesen Substanzen sind vorteilhaft zu verwenden.

Die Herstellung der erfindungsgemäßen Zubereitungen kann in der üblichen Weise durch Mischen der einzelnen Bestandteile erfolgen. Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure/Phosphat-Puffergemischen. In einer Ausführungsform der Erfindung liegt der pH-Wert unter 10, z.B. im Bereich von 2-7, insbesondere im Bereich von 3-5.

Bevorzugte Shampooformulierungen enthalten
a) 0,05 bis 10 Gew.-% wenigstens eines erfindungsgemäßen Fällungspolymerisats,
b) 25 bis 94,95 Gew.-% Wasser,
c) 5 bis 50 Gew.-% Tenside,
d) 0 bis 5 Gew.-% eines Konditioniermittels,
e) 0 bis 10 Gew.-% weitere kosmetische Bestandteile.

In einer weiteren Ausführungsform können durch den Einsatz der erfindungsgemäßen Fällungspolymerisate auch tensidreduzierte Formulierungen mit weniger als 10 Gew.-% Tensid, bezogen auf die Zubereitung, in einer für die Zubereitung ausreichenden Viskosität hergestellt werden. Insbesondere werden die rheologiemodifizierenden erfindungsgemäßen Fällungspolymerisate zur Einstellung der gewünschten Viskosität in solchen Zubereitungen eingesetzt, die mindestens 0,1 Gew.-% und von 0,1 bis 10 Gew.-%, bevorzugt weniger als 10 Gew.-% Tensid enthalten.

In den Shampoos und kosmetischen Reinigungsmitteln können alle in Shampoos und kosmetischen Reinigungsmitteln üblicherweise eingesetzten anionischen, neutralen, amphoteren oder kationischen Tenside verwendet werden. Geeignete Tenside wurden vorstehend genannt. Besonders bevorzugt sind Shampoos und kosmetische Reinigungsmittel mit einem Tensidgehalt von mehr als 10 Gew.-%.

In den Shampooformulierungen können zur Erzielung bestimmter Effekte weitere Konditioniermittel eingesetzt werden. Hierzu zählen beispielsweise kationische Polymere mit der INCI-Bezeichnung Polyquaternium, insbesondere Copolymere aus Vinylpyrrolidon/N-Vinylimidazoliumsalzen (Luviquat^{®}FC, Luviquat^{®}HM, Luviquat^{®}MS, Luviquat^{®}Care), Copolymere aus N-Vinylpyrrolidon/Dimethylaminoethylmethacrylat, quaternisiert mit Diethylsulfat (Luviquat^{®}PQ 11), Copolymere aus N-Vinylcaprolactam/N-Vinylpyrrolidon/N-Vinylimidazoliumsalzen (Luviquat^{®}Hold); kationische Cellulosederivate (Polyquaternium-4 und -10), Acrylamidcopolymere (Polyquaternium-7).

Vorteilhafte Konditionierungsmittel stellen beispielsweise die nach INCI als Polyquaternium bezeichneten Verbindungen dar (insbesondere Polyquaternium-1 bis Polyquaternium-87). Die folgende Tabelle gibt eine nicht abschließende Übersicht über in den erfindungsgemäßen Shampoos und Haarwaschmitteln verwendbaren Konditioniermittel:

| Bezeichnung nach INCI | CAS-Nummer | Polymertyp | Beispiel (Handelsname) |
|---|---|---|---|
| Polyquaternium-2 | CAS 63451-27-4 | Urea, N, N', bis [3-(dimethylamino)propyl]-polymer mit 1, 1'-oxybis (2-chloroethan) | Mirapol® A-15 |
| Polyquaternium-5 | CAS 26006-22-4 | Acrylamid, β-Methacryloxyethyltriethylammoniummethosulfat | |
| Polyquater nium-6 | CAS 26062-79-3 | N,N-Dimethyl-N-2-propenyl-2-propen-aminiumchlorid (PolyDADMAC) | Merquat® 100 |
| Polyquaternium-7 | CAS 26590-05-6 | N,N-Dimethyl-N-2-propenyl-2-propenaminiumchlorid, 2-Propenamid | Merquat® S |
| Polyquaternium-10 | CAS 53568-66-4,55353-19-0, 54351-50-7, 68610-92-4, 81859-24-7 | Quaternäres Ammoniumsalz der Hydroxyethylcellulose | Celquat® SC-230M, Polymer JR 400 |
| Polyquaternium-11 | CAS 53633-54-8 | Vinylpyrrolidon/dimethylaminoethyl-Methacrylat-Copolymer/Diethylsulfat-Reaktionsprodukt | Gafquat® 755N |
| Polyquaternium-16 | CAS 29297-55-0 | Vinylpyrrolidon/vinylimidazolinum-methochlorid-Copolymer | Luviquat® HM552 |
| Polyquaternium-17 | CAS 90624-75-2 | | Mirapol® AD-1 |
| Polyquaternium-19 | CAS 110736-85-1 | Quaternisierter wasserlöslicher Polyvinylalkohol | |
| Polyquaternium-20 | CAS 110736-86-2 | In Wasser dispergierbarer quaternisierter Polyvinylocatedecylether | |
| Polyquaternium-21 | | Polysiloxanpolydimethyldimethylammoniumacetat-Copolymer | Abil® B 9905 |
| Polyquaternium-22 | CAS 53694-17-0 | Dimethyldiallylammoniumchlo-rid/Acrylsäure-Copolymer | Merquat® 280 |
| Polyquaternium-24 | CAS 107897-23-5 | Polymeres quaternäres Ammoniumsalz der Hydroxyethylcellulose | Quartisoft® LM-200 |
| Polyquaternium-28 | CAS 131954-48-8 | Vinylpyrroldon/Methacrylamido-propyltrimethylammoniumchlorid-Copolymer | Gafquat® HS-100 |
| Polyquaternium-29 | CAS 92091-36-6, 148880-30-2 | Chitosan, das mit Propylenoxid umgesetzt u. mit Epichlorhydrin quaternisiert wurde | Lexquat ® CH |
| Polyquaternium-31 | CAS 136505-02-7, 139767-67-7 | Polymeres, quaternäres Ammoniumsalz, das durch die Umsetzung von DMAPA-Acrylate/Acrylsäure/ Acrylonitrogens-Copolymeren u. Diethylsulfat hergestellt wird | Hypan ® QT 100 |
| Polyquaternium-32 | CAS 35429-19-7 | N,N,N-Trimethyl-2- ([82-methyl-1-oxo-2-propenyl)oxy]-ethanaminium chlorid, polymer mit 2-Propenamid | |
| Polyqua-ternium-37 | CAS 26161-33-1 | | |
| Polyqua-ternium-44 | | Copolymeres quaternes Ammoniumsalz aus Vinylpyrrolidon und quaternisiertem Imidazolin | |
| Polyquaternium-67 | | polymeric quaternary ammonium salt of hydroxyethyl cellulose reacted with a trimethyl ammonium substituted epoxide and a lauryl dimethyl ammonium substituted epoxide | SoftCAT^{®} |
| Polyquaternium-74 | | | Poly-care^{®} Boost |
| Polyquaternium-87 | | | Luviquat^{®} Sensation |

Die erfindungsgemäßen Haargele werden in für Gele üblichen Behältern bereitgestellt, bevorzugt in Tuben oder Tiegeln.

### Beispiele

Die Erfindung wird durch die nachfolgenden Beispiele näher beschrieben aber nicht auf diese beschränkt.

Bedeutung der Abkürzungen/Handelsnamen:

| | |
|---|---|
| Acrylsäure | AS |
| Allyl-D-Gluconamid | AGA |
| Methacrylamid | MAM |
| Methylmethacrylat | MMA |
| N-Vinylimidazol | VI |
| N-Vinylpyrrolidon | VP |
| Pentaerythrittriallylether | PETAE |
| Ureidomethacrylat | UMA |
| Plex^{®} 6844-O | 25 Gew.-% UMA in MMA |
| Plex^{®} 6877-O | 25 Gew.-% C₁₆₋₁₈-Alkyl-(EO)₂₅-Methacrylat in MMA |
| AMP | 2-Amino-2-methylpropanol |

Sofern nicht ausdrücklich anders bestimmt, handelt es sich bei den Mengenangaben in "%" um Gewichts-Prozent-Angaben.

### Herstellung der Fällungspolymerisate

### Herstellungsvorschrift (A) für Beispiel 1

| | | |
|---|---|---|
| Vorlage: | Ethylacetat | 535 g |
| | Cyclohexan | 192 g |
| | Tert.-Butylperoctoat | 1,2 g |
| | Zulauf 1 | 57,3 g |
| | Zulauf 2 | 7 g |
| | | |
| Zulauf 1: | N-Vinylpyrrolidon | 235,2 g |
| | Methacrylamid | 4,8 g |
| | Ethylacetat | 46,5 g |
| | | |
| Zulauf 2: | Ethylacetat | 46,5 g |
| | Wako V65 | 0,2 g |
| | | |
| Zulauf 3: | Ethylacetat | 140 g |
| | Wako V65 | 0,75 g |
| | Tert.- Butylperoctoat | 1,2 g |

Die Vorlage wurde unter Stickstoffatmosphäre auf ca. 62°C erhitzt. Zulauf 1 wurde dann innerhalb von 3 Stunden und Zulauf 2 innerhalb von 4 Stunden zudosiert. Die Reaktionsmischung wurde dann 2 weitere Stunden unter Rühren bei ca. 62°C gehalten. Danach wurde Zulauf 3 während 30 Minuten zugegeben und bei 65°C für weitere 2,5 Stunden gerührt. Danach wurde zunächst auf 70°C erhitzt und 3 weitere Stunden polymerisiert und schließlich auf 90°C erhitzt und weitere 4 Stunden nachpolymerisiert. Die Reaktionsmischung ließ man dann auf Raumtemperatur abkühlen, der Feststoff wurde abfiltriert und 24 Stunden unter Vakuum bei 75°C getrocknet.

Die Herstellung der Polymere gemäß der Beispiele 2, 6, 7 und 12 wurde in analoger Weise durchgeführt.

### Herstellungsvorschrift (B) für Beispiel 4

| | | |
|---|---|---|
| Vorlage: | Ethylacetat | 702 g |
| | Tert.-Butylperoctoat | 2 g |
| | Zulauf 1 | 62 g |
| | Zulauf 2 | 7 g |
| | | |
| Zulauf 1: | N-Vinylpyrrolidon | 237,6 g |
| | Methacrylamid | 13,2 g |
| | 1-Vinylimidazol | 13,2 g |
| | Ethylacetat | 46,8 g |
| | | |
| Zulauf 2: | Ethylacetat | 46,8 g |
| | Wako^{®}V65 | 0,26 g |
| | | |
| Zulauf 3: | Ethylacetat | 140,4 g |
| | Wako^{®}V65 | 0,79 g |
| | Tert.- Butylperoctoat | 1,32 g |

### Die Vorlage wurde unter Stickstoffatmosphäre auf ca. 60°C erhitzt. Zulauf 1 wurde dann innerhalb von 3 Stunden und Zulauf 2 innerhalb von 4 Stunden zudosiert. Die Reaktionsmischung wurde dann 2 weitere Stunden unter Rühren bei ca. 60°C gehalten. Danach wurde Zulauf 3 während 30 Minuten zugegeben und bei 65°C für weitere 2,5 Stunden gerührt. Danach wurde zunächst auf 70°C erhitzt und 3 weitere Stunden polymerisiert und schließlich auf 80-85°C erhitzt und weitere 4 Stunden nachpolymerisiert. Die Reaktionsmischung ließ man dann auf Raumtemperatur abkühlen, der Feststoff wurde abfiltriert und 24 Stunden unter Vakuum bei 75°C getrocknet.

Die Herstellung der Polymere gemäß der Beispiele 3, 9, 10, 11, 13 und 14 wurde in analoger Weise durchgeführt.

### Herstellungsvorschrift (C) für Beispiel 5

| | | |
|---|---|---|
| Vorlage: | Ethylacetat | 535 g |
| | Cyclohexan | 192 g |
| | Tert.-Butylperoctoat | 1,2 g |
| | Zulauf 1 | 57,3 g |
| | Zulauf 2 | 7 g |
| | | |
| Zulauf 1: | N-Vinylpyrrolidon | 216 g |
| | Methacrylamid | 7,2 g |
| | Vinylimidazol | 16,3 g |
| | Pentaerythrittriallylether | 0,48 g |
| | Ethylacetat | 46,5 g |
| | | |
| Zulauf 2: | Ethylacetat | 46,5 g |
| | Wako V65 | 0,2 g |
| | | |
| Zulauf 3: | Ethylacetat | 140 g |
| | Wako V65 | 0,75 g |
| | Tert.- Butylperoctoat | 1,2 g |
| | | |
| Zulauf 4: | Methylchlorid (Gas) | 9 g |

Die Vorlage wurde unter Stickstoffatmosphäre auf ca. 62°C erhitzt. Zulauf 1 wurde dann innerhalb von 3 Stunden und Zulauf 2 innerhalb von 4 Stunden zudosiert. Die Reaktionsmischung wurde dann 2 weitere Stunden unter Rühren bei ca. 62°C gehalten. Danach wurde Zulauf 3 während 30 Minuten zugegeben und bei 65°C für weitere 2,5 Stunden gerührt. Danach wurde zunächst auf 70°C erhitzt und 3 weitere Stunden polymerisiert und schließlich auf 90°C erhitzt und weitere 4 Stunden nachpolymerisiert. Die Reaktionsmischung ließ man dann unter Rühren auf ca. 50°C abkühlen, Zulauf 4 wurde während ca. 30 Minuten einleitet. Das Polymer wurde dann für jeweils 1 Stunde bei 70°C und bei 90°C methyliert. Anschließend wurde nicht verbrauchtes Methylchlorid durch Entlüften entfernt. Der Feststoff wurde dann abfiltriert und 24 Stunden unter Vakuum bei 75°C getrocknet.

Die Herstellung des Polymers gemäß Beispiel 8 wurde in analoger Weise durchgeführt.

Die quaternisierbaren Gruppen der Polymere der Beispiele 5 und 8 werden zu ca. 75 mol-% quaternisiert.

### Erfindungsgemäße Fällungspolymerisate:

| Nr. | VP | MAM | Plex^{®} 6844-O | AGA | AS | VI | Plex^{®} 6877-O | PETAE | Typ |
|---|---|---|---|---|---|---|---|---|---|
| 1 | 98 | 2 | -- | -- | -- | -- | -- | -- | N1 |
| 2 | 95 | 5 | -- | -- | -- | -- | -- | -- | N2 |
| 3 | 90 | 3 | -- | -- | -- | 7 | -- | -- | K1 |
| 4 | 90 | 5 | -- | -- | -- | 5 | -- | -- | K2 |
| 5 | 90 | 3 | -- | -- | -- | 6,8 | -- | 0,2 | K3 |
| 6 | 80 | 10 | 6 | -- | -- | 4 | -- | -- | K4 |
| 7 | 88 | -- | 4 | -- | -- | 6 | 2 | -- | K5 |
| 8 | 80 | -- | -- | 1 | -- | 15 | 4 | -- | K6 |
| 9 | 95 | 3 | -- | -- | 2 | -- | -- | -- | A1 |
| 10 | 93 | 2 | -- | -- | 5 | -- | -- | -- | A2 |
| 11 | 92 | -- | 2 | -- | 6 | -- | -- | -- | A3 |
| 12 | 90 | -- | -- | 1 | 9 | -- | -- | -- | A6 |
| 13 | 86 | -- | 4 | -- | 10 | -- | -- | -- | A4 |
| 14 | 80 | -- | 4 | -- | 10 | -- | 6 | -- | A5 |

### Anwendungstechnische Beispiele

### Haargele

Alle Gele wurden mit 2-3 Gew.-% Festigerpolymer, 0.2 oder 0.5 Gew.-% Carbopol^{®}980 angesetzt und mit Triethanolamin auf pH ca. 7 eingestellt.
Zur Herstellung der Standardgele (SG1 bis SG3) wurden als Festigerpolymere Polyvinylpyrrolidon oder Vinylpyrrolidon-Vinylacetat-Copolymere verwendet.

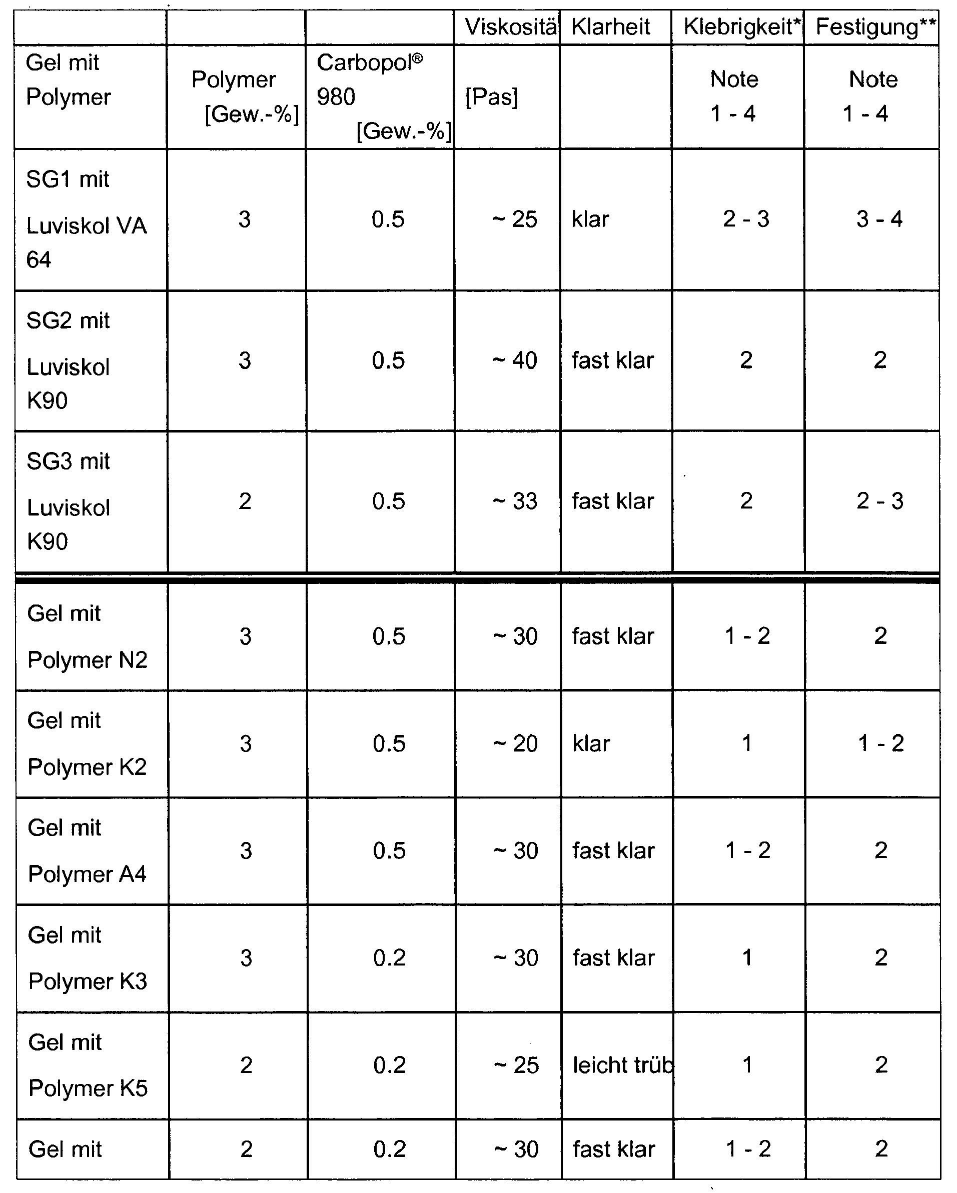

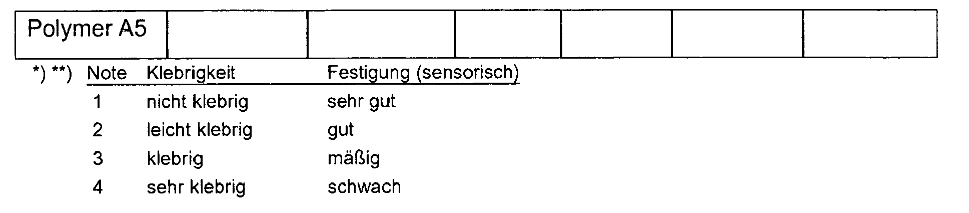

Im Folgenden sind beispielhafte kosmetische Zubereitungen dargestellt, die die erfindungsgemäßen Fällungspolymerisate enthalten.

### Haargele mit Carbopol^{®}

Verdickerphase (Phase 1):

| | |
|---|---|
| Carbopol^{®}940 (Pulver) | 1g |
| Wasser | 149 g |
| Euxyl^{®}K100 | q.s. |
| mit Triethanolamin (99%) | auf pH 6,5 - 7,2 einstellen |

### Festigerphase (Phase 2):

| | |
|---|---|
| Polymer 1 | 6 g |
| Cremophor^{®}WO : Parfüm [4:1 w/w] | 0,3 g |
| Wasser | mit Wasser auf 50 g auflösen |

Die Phasen 1 und 2 werden getrennt unter Rühren homogenisiert; man erhält ein klares, dickes Gel (Verdicker-Phase) und eine Festigerphase. Danach wird die Festigerphase langsam in die Verdickerphase eingerührt, so dass man ein nahezu klares, festes Gel erhält.

Analoge Haargele werden mit den Polymeren der Beispiele 2, 3, 4, 6, 11 und 13 hergestellt.

### Haargele mit PVP und Carbopol^{®}

### Verdickerphase (Phase 1):

| | |
|---|---|
| Carbopol^{®}940 (Pulver) | 1g |
| Wasser | 149 g |
| Euxyl^{®}K100 | q.s. |
| mit Triethanolamin (99%ig) | auf pH 6,5 - 7,2 einstellen |

### Festigerphase (Phase 2):

| | |
|---|---|
| Polymer 1 | 3 g |
| Polyvinylpyrrolidon PVP K90 | 3 g |
| Cremophor^{®}WO : Parfüm [4:1 w/w] | 0,3 g |
| Wasser | mit Wasser auf 50 g auflösen |

Die Phasen 1 und 2 werden getrennt unter Rühren homogenisiert; man erhält ein klares, dickes Gel (Verdickerphase) und eine Festigerphase. Danach wird die Festigerphase langsam in die Verdickerphase eingerührt, so dass man ein nahezu klares, festes Gel erhält.

Analoge Haargele werde mit den Polymeren der Beispiele 2, 3, 4, 6, 11 und 13 hergestellt.

### Haargele mit Assoziativverdicker

### Verdickerphase (Phase 1):

| | |
|---|---|
| Aculyne^{®}22 (30%) | 6 g |
| Wasser | 144 g |
| Euxyl^{®}K100 | q.s. |
| mit AMP (90%ig) | auf pH 6,5 - 7,2 einstellen |

### Festigerphase (Phase 2):

| | |
|---|---|
| Polymer 1 | 6 g |
| Cremophor^{®}WO : Parfüm [4:1 w/w] | 0,3 g |
| Wasser | auf 50 g auflösend verdünnen |

Die Phasen 1 und 2 werden getrennt unter Rühren homogenisiert; man erhält ein klares, dickes Gel (Verdickerphase) und eine Festigerphase. Danach wird die Festigerphase langsam in die Verdickerphase eingerührt, so dass man ein nahezu klares, festes Gel erhält.

Analoge Haargele werde mit den Polymeren der Beispiele 2, 3, 4, 5, 6, 7, 8, 9, 10, 11, 12,13 und 14 hergestellt.

### Haargele mit Assoziativverdicker und kationischem Festigerpolymer

### Verdickerphase (Phase 1):

| | |
|---|---|
| Aculyne 22 (30%ig) | 6 g |
| Wasser | 144 g |
| Euxyl^{®}K100 | q.s. |
| mit AMP (90%ig) | auf pH 6,5 - 7,2 einstellen |

### Festigerphase (Phase 2):

| | |
|---|---|
| Polymer 1 | 4 g |
| Luviquat^{®}Supreme (20% ig) | 5 g |
| Cremophor WO : Parfüm [4:1 w/w] | 0,3 g |
| Wasser | auf 50 g auflösend verdünnen |

Die Phasen 1 und 2 werden getrennt unter Rühren homogenisiert; man erhält ein klares, dickes Gel (Verdickerphase) und eine Festigerphase. Danach wird die Festigerphase langsam in die Verdickerphase eingerührt, so dass man ein nahezu klares, festes Gel erhält.

Analoge Haargele werden mit den Polymeren der Beispiele 2, 3, 4, 5, 6, 7, 8, 9, 10, 11, 12,13 und 14 hergestellt.

### Haargele mit Assoziativverdicker und anionischem Festigerpolymer

### Verdickerphase (Phase 1):

| | |
|---|---|
| Aculyne 22 (30%ige) | 6 g |
| Wasser | 144 g |
| K100 | q.s. |
| mit AMP (90%ig) | auf pH 6,5 - 7,2 einstellen |

### Festigerphase (Phase 2):

| | |
|---|---|
| Polymer 1 | 3 g |
| Ultrahold^{®}Strong (100%) | 3 g |
| Cremophor WO : Parfüm [4:1 w/w] | 0,3 g |

Mit Wasser auf 50 g verdünnen, dann mit AMP auf pH 7,5 bis 8 einstellen.

Die Phasen 1 und 2 werden getrennt unter Rühren homogenisiert; man erhält ein klares, dickes Gel (Verdickerphase) und eine Festigerphase. Danach wird die Festigerphase langsam in die Verdickerphase eingerührt, so dass man ein nahezu klares, festes Gel erhält.

Analoge Haargele werden mit den Polymeren der Beispiele 2, 9, 10, 11,12,13 und 14 hergestellt.

| Schaumfestiger | [Gew.-%] |
|---|---|
| Polymer 3 (Pulver) | 1,00 |
| Cremophor^{®}A 25 (Ceteareth 25/ BASF) | 0,2 |
| Comperlan^{®}KD (Coamide DEA / Henkel) | 0,1 |
| Wasser | 78,7 |
| Mit Milchsäure (90%) auf pH 5,5 bis 6,5 einstellen | |
| Dimethylether | 10,0 |

### Weitere Zusatz: Parfüm, Konservierungsmittel

### Herstellung : Einwiegen und unter Rühren lösen, Abfüllen und Treibgas zusetzen.

Analoge Schaumfestiger werden mit den Polymeren der Beispiele 4, 5, 6, 7 und 8 hergestellt.

| Shampoos | Gew.-% |
|---|---|
| | |
| A) Texapon^{®}NSO 28%ig | 50,0 |
| Comperlan^{®} KD | 1,0 |
| Polymer 5 | 1,0 |
| Wasser | 19,0 |
| Parfümöl | q.s. |
| | |
| B) Wasser | 28,0 |
| Natriumchlorid | 1,0 |
| Konservierungsmittel | q.s. |

Herstellung:'Einwiegen und unter Rühren Phasen A und B getrennt lösen und mischen, Phase B langsam in Phase A einrühren.

Analoge Shampoos werden mit den Polymeren der Beispiele 6, 7 und 8 hergestellt.

### Hautkosmetische Zubereitungen

### Standard O/W Creme

| Ölphase : | Gew.-% | CTFA |
|---|---|---|
| Cremophor^{®}A6 | 3,3 | Ceteareth-6 (and) Stearyl Alkohol |
| Cremophor^{®}A25 | 3,3 | Ceteareth-25 |
| Glycerinmonostearat s.e. | 2,5 | Glyceryl Stearate |
| Paraffinöl | 7,5 | Paraffin Oil |
| Cetylalkohol | 2,5 | Cetyl Alkohol |
| Luvitol^{®}EHO | 3,2 | CetearylOctanoate |
| Vitamin-E-acetate | 1,0 | Tocopheryl Acetate |
| Nip-Nip | 0,1 | Methyl- und Propyl-4-hydroxybenzoate (7:3) |

| Wasserphase: | Gew.-% | |
|---|---|---|
| Polymer 5 | 1,0 | |
| Wasser | 74,0 | Water |
| 1,2- Propylenglykol | 1,5 | Propylene Glycol |
| Germall II | 0,1 | Imidazolidinyl-Urea |

### Herstellung :

Einwiegen und unter Rühren Ölphase und Wasserphase getrennt bei einer Temperatur von ca. 80°C homogenisieren; Wasserphase langsam in Ölphase einrühren; unter Rühren langsam auf Raumtemperatur abkühlen.

Analoge O/W-Cremes werden mit den Polymeren der Beispiele 6, 7, 8,11, 13 und 14 hergestellt. Bei den Polymeren 5, 6, 7 und 8 wird die Wasserphase mit Milchsäure auf pH 5-6, bei den Polymeren 11, 13 und 14 mit Triethanolamin auf pH 6,5 bis 7,2 eingestellt.

### Standard-Tageslotion

| Ölphase : | % | CTFA Name |
|---|---|---|
| | | |
| Cremophor^{®}A6 | 1,5 | Ceteareth-6 (and) Stearyl Alkohol |
| Cremophor^{®}A25 | 1,5 | Ceteareth-25 |
| Glycerinmonostearat s.e. | 5,0 | Glyceryl Stearate |
| Uvinul^{®}MS 40 | 0,5 | Bezophenone-4 |
| Paraffinöl | 3,5 | Paraffin Oil |
| Cetylalkohol | 0,5 | Cetyl Alkohol |
| Luvitol^{®}EHO | 10,0 | Cetearyl Octanoate |
| D-Panthenol 50 P | 3,0 | Panthenol und Propylenglykol |
| Vitamin-E-acetate | 1,0 | Tocopheryl Acetate |
| Tegiloxan^{®}100 | 0,3 | Dimethicon |
| Nip-Nip | 0,1 | Methyl- und Propyl-4-hydroxybenzoate (7:3) |
| | | |

| Wasserphase: | | |
|---|---|---|
| | | |
| Polymer 5 | 0,5 | |
| Wasser | 71,0 | Water |
| 1,2- Propylenglykol | 1,5 | Propylene Glycol |
| Germall II | 0,1 | Imidazolidinyl-Urea |

### Herstellung :

Einwiegen und unter Rühren Ölphase und Wasserphase getrennt bei einer Temperatur von ca. 80°C homogenisieren; Wasserphase langsam in Ölphase einrühren; unter Rühren langsam auf Raumtemperatur abkühlen.

Analoge Lotionen werden mit den Polymeren der Beispiele 6, 7 und 8 hergestellt.

## Patentansprüche

1. Polymerisate erhältlich durch Fällungspolymerisation, die
a) 80 bis 99,9 Gew.-% wenigstens eines nichtionischen wasserlöslichen Monomers a) ausgewählt aus N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam
und
b) 0,1 bis 20 Gew.-% eines Monomers ausgewählt aus
b1) Methacrylamid,
b2) radikalisch polymerisierbaren Verbindungen, die ein Strukturelement der Formel b2) enthalten 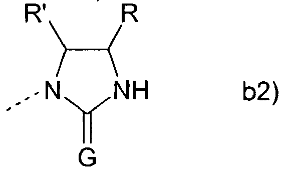 worin R und R' unabhängig voneinander Wasserstoff, Alkyl, Cycloalkyl, Heterocycloalkyl, Aryl oder Hetaryl, wobei R und R' auch kovalent verbunden sein können, und G für O oder NH steht,
b3) wenigstens eine Allylaminogruppe tragenden Polyhydroxyverbindungen und b4) deren Mischungen einpolymerisiert enthalten,
wobei die Gesamtmenge aller einpolymerisierten Monomere 100 Gew.-% beträgt.

2. Polymerisate nach Anspruch 1, wobei die Polymerisate a) N-Vinylpyrrolidon einpolymerisiert enthalten.

3. Polymerisate nach einem der Ansprüche 1 oder 2, wobei die Polymerisate von 0,5 bis 15 Gew.-% weitere, von a) und b) verschiedene Monomere c) einpolymerisiert enthalten, wobei die Gesamtmenge aller einpolymerisierten Monomere 100 Gew.-% beträgt.

4. Polymerisate nach einem der Ansprüche 1 bis 3, wobei die Polymerisate als ein Monomer c2) N-Vinylimidazol einpolymerisiert enthalten.

5. Polymerisate nach einem der Ansprüche 1 bis 4, wobei die Polymerisate als ein Monomer c1) Acrylsäure einpolymerisiert enthalten.

6. Polymerisate nach einem der Ansprüche 1 bis 5, wobei die Polymerisate als Monomer a) N-Vinylpyrrolidon und als Monomere c) c1) Acrylsäure und eines oder mehrere ausgewählt aus c2) N-Vinylimidazol, c3) DMAEMA und c4) DMAPMAM einpolymerisiert enthalten.

7. Polymerisate nach Anspruch 6, wobei das Gewichtsverhältnis von c1) zur Summe aus c2), c3) und c4) kleiner oder gleich 1:2 oder größer oder gleich 2:1 ist.

8. Polymerisate nach einem der Ansprüche 1 bis 7, wobei die Polymerisate als ein Monomer b) Methacrylamid einpolymerisiert enthalten.

9. Polymerisate nach einem der Ansprüche 1 bis 8, wobei die Polymerisate als ein Monomer b) Ureidomethacrylat einpolymerisiert enthalten.

10. Polymerisate nach einem der Ansprüche 1 bis 9, wobei die Polymerisate wenigstens einen Vernetzer einpolymerisiert enthalten.

11. Haarkosmetische Zubereitungen enthaltend wenigstens ein Fällungspolymerisat gemäß einem der Ansprüche 1 bis 10.

12. Verfahren zur Behandlung von Haaren, **dadurch gekennzeichnet, dass** die Haare mit einem Fällungspolymerisat gemäß einem der Ansprüche 1 bis 10 in Kontakt gebracht werden.

13. Verfahren zur Herstellung von Polymerisaten gemäß wenigstens eines der Ansprüche 1 bis 10, **dadurch gekennzeichnet, dass** das Verfahren eine Fällungspolymerisation umfasst.

## Claims

1. A polymer obtainable by precipitation polymerization which comprises, in copolymerized form,
a) 80 to 99.9% by weight of at least one nonionic water-soluble monomer a) selected from N-vinylpyrrolidone, N-vinylpiperidone, N-vinylcaprolactam, N-vinyl-5-methyl-2-pyrrolidone, N-vinyl-5-ethyl-2-pyrrolidone, N-vinyl-6-methyl-2-piperidone, N-vinyl-6-ethyl-2-piperidone, N-vinyl-7-methyl-2-caprolactam and N-vinyl-7-ethyl-2-caprolactam
and
b) 0.1 to 20% by weight of a monomer selected from
b1) methacrylamide,
b2) radically polymerizable compounds which comprise a structural element of the formula b2) 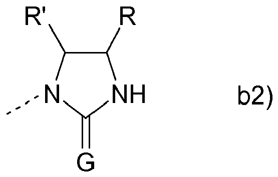 in which R and R', independently of one another, are hydrogen, alkyl, cycloalkyl, heterocycloalkyl, aryl or hetaryl, where R and R' may also be covalently bonded, and G is 0 or NH,
b3) polyhydroxy compounds carrying at least one allylamino group and
b4) mixtures thereof,
where the total amount of all of the copolymerized monomers is 100% by weight.

2. The polymer according to claim 1, where the polymer comprises a) N-vinylpyrrolidone in copolymerized form.

3. The polymer according to either of claims 1 and 2, where the polymer comprises from 0.5 to 15% by weight of further monomers c) different from a) and b) in copolymerized form, where the total amount of all of the copolymerized monomers is 100% by weight.

4. The polymer according to any of claims 1 to 3, where the polymer comprises N-vinylimidazole in copolymerized form as a monomer c2).

5. The polymer according to any of claims 1 to 4, where the polymer comprises acrylic acid in copolymerized form as a monomer c1).

6. The polymer according to any of claims 1 to 5, where the polymer comprises, in copolymerized form, as monomer a), N-vinylpyrrolidone and, as monomer c), c1) acrylic acid and one or more selected from c2) N-vinylimidazole, c3) DMAEMA and c4) DMAPMAM.

7. The polymer according to claim 6, where the weight ratio of c1) to the sum of c2), c3) and c4) is less than or equal to 1:2 or greater than or equal to 2:1.

8. The polymer according to any of claims 1 to 7, where the polymer comprises methacrylamide in copolymerized form as a monomer b).

9. The polymer according to any of claims 1 to 8, where the polymer comprises ureidomethacrylate in copolymerized form as a monomer b).

10. The polymer according to any of claims 1 to 9, where the polymer comprises at least one crosslinker in copolymerized form.

11. A hair cosmetic preparation comprising at least one precipitation polymer according to any of claims 1 to 10.

12. A method of treating hair, wherein the hair is brought into contact with a precipitation polymer according to any of claims 1 to 10.

13. A method of preparing polymers according to at least one of claims 1 to 10, wherein the method comprises a precipitation polymerization.

## Revendications

1. Produits de polymérisation pouvant être obtenus par polymérisation par précipitation, qui contiennent incorporés par polymérisation
a) 80 à 99,9 % en poids d'au moins un monomère a) non ionique hydrosoluble, choisi parmi la N-vinylpyrrolidone, la N-vinylpipéridone, le N-vinyl-caprolactame, la N-vinyl-5-méthyl-2-pyrrolidone, la N-vinyl-5-éthyl-2-pyrrolidone, la N-vinyl-6-méthyl-2-pipéridone, la N-vinyl-6-éthyl-2-pipéridone, le N-vinyl-7-méthyl-2-caprolactame, le N-vinyl-7-éthyl-2-caprolactame,
et
b) 0,1 à 20 % en poids d'un monomère choisi parmi
b1) le méthacrylamide,
b2) des composés polymérisables par voie radicalaire, qui contiennent un élément structural de formule b2) 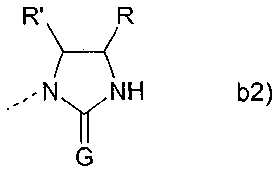 dans laquelle R et R' représentent indépendamment l'un de l'autre un atome d'hydrogène, un groupe alkyle, cycloalkyle, hétérocycloalkyle, aryle ou hétéroaryle, R et R' pouvant également être liés par covalence, et G représente 0 ou NH,
b3) des composés polyhydroxylés portant au moins un groupe alkylamino et
b4) des mélanges de ceux-ci,
la quantité totale de tous les monomères incorporés par polymérisation étant égale à 100 % en poids.

2. Produits de polymérisation selon la revendication 1, les produits de polymérisation contenant incorporée par polymérisation a) de la N-vinylpyrrolidone.

3. Produits de polymérisation selon l'une quelconque des revendications 1 et 2, les produits de polymérisation contenant incorporés par polymérisation de 0,5 à 15 % en poids d'autres monomères c) différents de a) et b), la quantité totale de tous les monomères incorporés par polymérisation étant égale à 100 % en poids.

4. Produits de polymérisation selon l'une quelconque des revendications 1 à 3, les produits de polymérisation contenant incorporé par polymérisation en tant qu'un monomère c2) du N-vinylimidazole.

5. Produits de polymérisation selon l'une quelconque des revendications 1 à 4, les produits de polymérisation contenant incorporé par polymérisation en tant qu'un monomère c1) de l'acide acrylique.

6. Produits de polymérisation selon l'une quelconque des revendications 1 à 5, les produits de polymérisation contenant incorporés par polymérisation en tant que monomère a) de la N-vinylpyrrolidone et en tant que monomères c) c1) de l'acide acrylique et un ou plusieurs monomères choisis parmi c2) le N-vinylimidazole, c3) le DMAEMA et c4) le DMAPMAM.

7. Produits de polymérisation selon la revendication 6, dans lesquels le rapport pondéral de c1) à la somme de c2), c3) et c4) est inférieur ou égal à 1:2 ou supérieur ou égal à 2:1.

8. Produits de polymérisation selon l'une quelconque des revendications 1 à 7, les produits de polymérisation contenant incorporé par polymérisation en tant qu'un monomère b) du méthacrylamide.

9. Produits de polymérisation selon l'une quelconque des revendications 1 à 8, les produits de polymérisation contenant incorporé par polymérisation en tant qu'un monomère b) de l'uréidométhacrylate.

10. Produits de polymérisation selon l'une quelconque des revendications 1 à 9, les produits de polymérisation contenant incorporé par polymérisation au moins un agent de réticulation.

11. Préparations cosmétiques capillaires contenant au moins un produit de polymérisation par précipitation selon l'une quelconque des revendications 1 à 10.

12. Procédé pour le traitement des cheveux, **caractérisé en ce qu'**on met les cheveux en contact avec un produit de polymérisation en émulsion selon l'une quelconque des revendications 1 à 10.

13. Procédé pour la préparation de produits de polymérisation selon au moins l'une quelconque des revendications 1 à 10, **caractérisé en ce que** le procédé comprend une polymérisation par précipitation.
